# EUROPEAN PATENT SPECIFICATION

(11) **EP 2 925 413 B1**
(45) Date of publication and mention of the grant of the patent: **03.07.2019**
(21) Application number: 13802305.6
(22) Date of filing: 29.11.2013
(51) Int. Cl.: A61Q 15/00, A61K 8/06, A61K 8/81, A61K 8/04

(54) **COSMETIC WATER-IN-OIL EMULSION IN AEROSOL FORM, COMPRISING AT LEAST ONE VINYL POLYMER CONTAINING AT LEAST ONE CARBOSILOXANE DENDRIMER-BASED UNIT, AT LEAST ONE OLEFIN COPOLYMER AND AT LEAST ONE ANTIPERSPIRANT ACTIVE AGENT**
KOSMETISCHE WASSER-IN-ÖL EMULSION ALS AEROSOL, ENTHALTEND WENIGSTENS EIN VINYLPOLYMER MIT ZUMINDEST EINEM MOTIF BASIEREND AUF EINEM CARBOSILOXAN-DENDRIMER, WENIGSTENS EIN OLEFINISCHES COPOLYMER UND WENIGSTENS EINEN ANTITRANSPIRANTIEN-WIRKSTOFF
COMPOSITION COSMÉTIQUE SOUS FORME D'ÉMULSION EAU-DANS-HUILE SOUS FORME D'AÉROSOL, COMPRENANT AU MOINS UN POLYMÈRE VINYLIQUE AYANT AU MOINS UN MOTIF DÉRIVÉ DE DENDRIMÈRE CARBOSILOXANE, AU MOINS UN COPOLYMÈRE D'OLÉFINE ET AU MOINS UN ACTIF ANTITRANSPIRANT

(30) Priority: 30.11.2012 FR 1261481; 20.12.2012 US 201261740116 P
(43) Date of publication of application: 07.10.2015
(73) Proprietor: L'Oréal, 75008 Paris (FR)
(72) Inventor: AUBRUN, Odile, F-92160 Antony (FR); SEBILLOTTE-ARNAUD, Laurence, F-94240 L'hay Les Roses (FR); JALENQUES, Xavier, F-92230 Gennevilliers (FR)
(74) Representative: Casalonga
(86) International application number: PCT/EP2013/075153
(87) International publication number: WO 2014/083175

(56) References cited:
- EP-A1- 1 034 776
- EP-A1- 1 586 304
- EP-A1- 1 862 162
- EP-A1- 2 492 301
- GB-A- 2 400 555
- US-A1- 2005 002 883

## Description

The present invention relates to an antiperspirant cosmetic composition in water-in-oil emulsion form, packaged in aerosol form, comprising, in a cosmetically acceptable medium, at least one antiperspirant active agent, at least one vinyl polymer containing at least one carbosiloxane dendrimer-based unit, and at least one olefin copolymer.

The invention also relates to a process for cosmetic treatment of human perspiration, and optionally of underarm odour, consisting in applying an effective amount of said composition to the surface of the skin.

The armpits and also certain other parts of the body are generally the site of much discomfort that may arise directly or indirectly from perspiration. This perspiration often leads to unpleasant and disagreeable sensations that are mainly due to the presence of sweat resulting from perspiration, which may, in certain cases, make the skin damp and clothing wet, in particular in the region of the armpits or of the back, thus leaving visible marks. Moreover, the presence of sweat most commonly gives rise to the production of body odour, which most of the time is unpleasant. Finally, during its evaporation, sweat may also leave salts and/or proteins on the surface of the skin, thus resulting in whitish marks on clothing. Such discomfort is noticed, including in the case of moderate perspiration.

In the cosmetics field, it is thus well known to use, in topical application, antiperspirant compositions containing substances that have the effect of limiting or even preventing the flow of sweat in order to overcome the problems mentioned above. These products are generally available in the form of roll-ons, sticks, aerosols or sprays.

Antiperspirant substances are generally formed from aluminium salts, such as aluminium chloride and aluminium hydroxyhalides, or complexes of aluminium and zirconium. These substances make it possible to reduce the flow of sweat by forming a plug in the sweat duct.

Antiperspirant compositions generally have the drawback of transferring when they are applied to the skin, i.e. the compositions are at least partly deposited, leaving marks, on certain supports with which they may be brought into contact, and in particular a piece of clothing. This results in the appearance of unsightly whitish marks on clothing and also to a loss of effectiveness of the antiperspirant compositions owing to their mediocre wear property on the skin. In particular, the appearance of these unsightly marks on clothing can discourage consumers from using antiperspirant compositions of this type.

EP1862162A1 discloses cosmetic compositions comprising a vinyl polymer and an olefin copolymer. EP2492301A1 discloses co-modified organopolysiloxanes and cosmetic compositions comprising them.

Thus, there is also a real need to use antiperspirant cosmetic compositions which do not have the abovementioned drawbacks, i.e. which result in a transfer-resistant deposit not leaving marks on the supports with which said compositions are brought into contact, such as clothing or fabrics, while at the same time maintaining a satisfactory antiperspirant efficiency.

The applicant has discovered, surprisingly, that, by using on the skin a cosmetic composition in water-in-oil emulsion form packaged in aerosol form, comprising one or more antiperspirant active agents, one or more vinyl polymers containing at least one carbosiloxane dendrimer-based unit, and one or more olefin copolymers, it is possible both to minimize or even prevent the appearance of unsightly whitish marks on the supports with which said composition is in contact, in particular clothing and fabrics of any type, and to produce a satisfactory antiperspirant effect.

The cosmetic composition thus used forms, at the time it is applied to the skin, a transfer-resistant antiperspirant film.

Moreover, the cosmetic composition in accordance with the invention is particularly mild and non-tacky, which is in particular favourable for underarm use. The film thus left behind provides a feeling of protection on the skin.

The subject of the present invention is therefore in particular an antiperspirant cosmetic composition in water-in-oil emulsion form, comprising, in a cosmetically acceptable medium, one or more antiperspirant active agents, one or more vinyl polymers containing at least one carbosiloxane dendrimer-based unit, and one or more olefin copolymers, said antiperspirant cosmetic composition being in an aerosol form.

The cosmetic composition in accordance with the invention results in a satisfactory antiperspirant effect and in the formation of a transfer-resistant deposit.

Moreover, the present invention also relates to a process for cosmetic treatment of human perspiration, and optionally of underarm odour, consisting in applying an effective amount of the composition as described previously to the surface of the skin.

In particular, the process according to the invention is in particular advantageous for treating the armpits, since the composition is not tacky and provides a non-oily feeling of softness.

The invention also relates to the use of said composition for providing a feeling of protection on the skin.

Other subjects, characteristics, aspects and advantages of the invention will emerge even more clearly on reading the description and the examples that follow.

The concentrations of starting materials or of active material which are given below are understood to be for the non-pressurized formulae. When the formula is pressurized, the concentration of starting material or of active material will be proportional to the amount of formula without propellant introduced into the aerosol device.

### Antiperspirant active agents

For the purpose of the present invention, the expression "antiperspirant active agent" is intended to mean any aluminium salt or complex which, by itself, has the effect of reducing the flow of sweat, of reducing the sensation on the skin of moisture associated with human sweat or of masking human sweat.

The antiperspirant active agents used in the composition according to the invention can be chosen from aluminium and/or zirconium salts; complexes of zirconium hydroxychloride and of aluminium hydroxychloride with an amino acid, such as those described in patent US-3 792 068, commonly known as "ZAG complexes". Such complexes are generally known under the name ZAG (when the amino acid is glycine). ZAG complexes ordinarily have an Al/Zr ratio ranging from about 1.67 to 12.5 and a metal/Cl ratio ranging from about 0.73 to 1.93. Among these products, mention may be made of aluminium zirconium octachlorohydrex GLY, aluminium zirconium pentachlorohydrex GLY, aluminium zirconium tetrachlorohydrate GLY and aluminium zirconium trichlorohydrate GLY.

Preferably, the antiperspirant active agents are chosen from aluminium salts.

Among the aluminium salts that may be mentioned are aluminium chlorohydrate, aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG, alum salts, aluminium sulfate, aluminium zirconium octachlorohydrate, aluminium zirconium pentachlorohydrate, aluminium zirconium tetrachlorohydrate, aluminium zirconium trichlorohydrate and more particularly the aluminium chlorohydrate sold by the company Reheis under the name Microdry aluminum Chlorohydrate or by the company Guilini Chemie under the name Aloxicoll PF 40. Aluminium salts and zirconium salts are for example the product sold by the company Reheis under the name Reach AZP-908-SUF®, or "activated" aluminium salts, for example the product sold by the company Reheis under the name Reach 103 or by the company Westwood under the name Westchlor 200.

Preferably, the antiperspirant active agent is aluminium hydrochloride.

The antiperspirant active agent(s) may be present in a content ranging from 0.5% to 25% by weight, preferably in a content ranging from 3% to 20% by weight and more preferentially in a content ranging from 3% to 15% by weight relative to the total weight of the composition.

### Vinyl polymer containing a carbosiloxane dendrimer

As indicated above, the cosmetic composition in accordance with the present invention comprises one or more vinyl polymers containing at least one carbosiloxane dendrimer-based unit.

The vinyl polymer may have in particular a backbone and at least one side chain, which comprises a carbosiloxane dendrimer-based unit containing a carbosiloxane dendrimer structure.

The term "carbosiloxane dendrimer structure" in the context of the present invention represents a molecular structure with branched groups of high molecular weights, said structure having high regularity in the radial direction starting from the bond to the backbone. Such carbosiloxane dendrimer structures are described in the form of a highly branched siloxane-silylalkylene copolymer in the laid-open Japanese patent application Kokai 9-171 154.

In accordance with a first embodiment, the vinyl polymer according to the invention may contain carbosiloxane dendrimer-based units that may be represented by the following general formula (I): 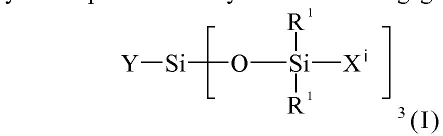 in which R¹ represents an aryl group or an alkyl group containing from 1 to 10 carbon atoms, and Xⁱ represents a silylalkyl group which, when i = 1, corresponds to formula (IA): 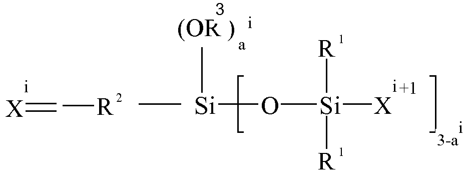 in which:
R¹ is as defined above, R² represents an alkylene group containing from 2 to 10 carbon atoms, R³ represents an alkyl group containing from 1 to 10 carbon atoms, Xⁱ⁺¹ represents a hydrogen atom, an alkyl group containing from 1 to 10 carbon atoms, an aryl group or the silylalkyl group as defined above with i = i + 1; i is an integer ranging from 1 to 10 indicating the generation of the starting silylalkyl group in each carbosiloxane dendritic structure with a value of 1 for the group Xⁱ in formual (IA), and aⁱ is an integer ranging from 0 to 3; Y represents a radical-polymerizable organic group chosen from:
- organic groups containing a methacrylic group or an acrylic group and that are represented by the formulae: 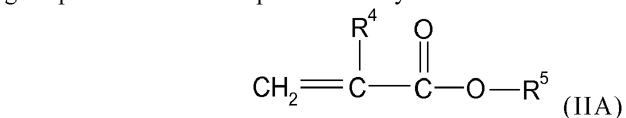 and 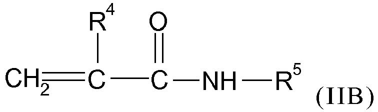 in which R⁴ represents a hydrogen atom or an alkyl group, R⁵ represents an alkylene group containing from 1 to 10 carbon atoms, such as a methylene group, an ethylene group, a propylene group or a butylene group, the methylene group and the propylene group being preferred; and
- organic groups containing a styryl group and that are represented by the formula: 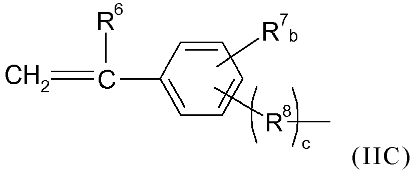 in which R⁶ represents a hydrogen atom or an alkyl group, R⁷ represents an alkyl group containing from 1 to 10 carbon atoms, such as a methyl group, an ethyl group, a propyl group or a butyl group, the methyl group being preferred, R⁸ represents an alkylene group containing from 1 to 10 carbon atoms, such as a methylene group, an ethylene group, a propylene group or a butylene group, the ethylene group being preferred, b is an integer ranging from 0 to 4, and c is 0 or 1 such that if c is 0, -(R⁸)_{c}- represents a bond.

According to one embodiment, R¹ may represent an alkyl group containing from 1 to 10 carbon atoms.

Preferably, R¹ represents an alkyl group chosen from a methyl group, an ethyl group, a propyl group, a butyl group, a pentyl group, an isopropyl group, an isobutyl group, a cyclopentyl group and a cyclohexyl group.

According to one embodiment, R¹ may represent an aryl group.

Preferably, the aryl group may be a phenyl group or a naphthyl group.

Preferably, R₁ represents a methyl or phenyl group and more preferentially a methyl group.

The vinyl polymer containing at least one carbosiloxane dendrimer-based unit has a molecular side chain containing a carbosiloxane dendrimer structure, and may be the product of polymerization of:
- from 0 to 99.9 parts by weight of a vinyl monomer (A) not containing fluoro organic groups; and
- from 100 to 0.1 parts by weight of a carbosiloxane dendrimer (B) containing a radical-polymerizable organic group, represented by general formula (I) as described above.

The monomer of vinyl type (A) is a monomer of vinyl type that contains a radical-polymerizable vinyl group.

Among the monomers of vinyl type, mention may be made of methyl methacrylate, ethyl methacrylate, n-propyl methacrylate, isopropyl methacrylate or a methacrylate of a lower alkyl analogue; glycidyl methacrylate; butyl methacrylate, butyl acrylate, n-butyl methacrylate, isobutyl methacrylate, tert-butyl acrylate, tert-butyl methacrylate, n-hexyl methacrylate, cyclohexyl methacrylate, 2-ethylhexyl acrylate, 2-ethylhexyl methacrylate, octyl methacrylate, lauryl methacrylate, stearyl acrylate, stearyl methacrylate or a higher-analogue methacrylate; or a higher-analogue methacrylate; vinyl acetate, vinyl propionate or a vinyl ester of a lower fatty acid analogue; vinyl caproate, vinyl 2-ethylhexoate, vinyl laurate, vinyl stearate or an ester of a higher fatty acid analogue; styrene, vinyltoluene, benzyl methacrylate, phenoxyethyl methacrylate, vinylpyrrolidone or similar vinylaromatic monomers; methacrylamide, N-methylolmethacrylamide, N-methoxymethylmethacrylamide, isobutoxymethoxymethacrylamide, N,N-dimethylmethacrylamide or similar monomers of vinyl type containing amide groups; hydroxyethyl methacrylate, hydroxypropyl alcohol methacrylate or similar monomers of vinyl type containing hydroxyl groups; acrylic acid, methacrylic acid, itaconic acid, crotonic acid, fumaric acid, maleic acid or similar monomers of vinyl type containing a carboxylic acid group; tetrahydrofurfuryl methacrylate, butoxyethyl methacrylate, ethoxydiethylene glycol methacrylate, polyethylene glycol methacrylate, polypropylene glycol monomethacrylate, hydroxybutyl vinyl ether, cetyl vinyl ether, 2-ethylhexyl vinyl ether or a similar monomer of vinyl type with ether bonds; methacryloxypropyltrimethoxysilane, polydimethylsiloxane containing a methacrylic group on one of its molecular ends, polydimethylsiloxane containing a styryl group on one of its molecular ends, or a similar silicone compound containing unsaturated groups; butadiene; vinyl chloride; vinylidene chloride; methacrylonitrile; dibutyl fumarate; anhydrous maleic acid; anhydrous succinic acid; methacryl glycidyl ether; an organic salt of an amine, an ammonium salt, and an alkali metal salt of methacrylic acid, of itaconic acid, of crotonic acid, of maleic acid or of fumaric acid; a radical-polymerizable unsaturated monomer containing a sulfonic acid group such as a styrenesulfonic acid group; a quaternary ammonium salt derived from methacrylic acid, such as 2-hydroxy-3-methacryloxypropyltrimethylammonium chloride; and a methacrylic acid ester of an alcohol containing a tertiary amine group, such as a methacrylic acid ester of diethylamine.

Multifunctional vinyl monomers may also be used.

Among the multifunctional vinyl monomers, mention may be made of trimethylolpropane trimethacrylate, pentaerythrityl trimethacrylate, ethylene glycol dimethacrylate, tetraethylene glycol dimethacrylate, polyethylene glycol dimethacrylate, 1,4-butanediol dimethacrylate, 1,6-hexanediol dimethacrylate, neopentyl glycol dimethacrylate, trimethylolpropanetrioxyethyl methacrylate, tris(2-hydroxyethyl)isocyanurate dimethacrylate, tris(2-hydroxyethyl)isocyanurate trimethacrylate, polydimethylsiloxane capped with styryl groups containing divinylbenzene groups on both ends, or similar silicone compounds containing unsaturated groups.

Preferably, the carbosiloxanne dendrimer (B) can be represented by the following formula: 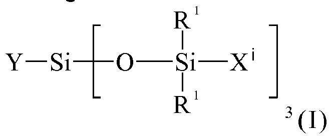 in which:
Y is a radical-polymerizable organic group chosen from an acryloxymethyl group, a 3-acryloxypropyl group, a methacryloxymethyl group, a 3-methacryloxypropyl group, a 4-vinylphenyl group, a 3-vinylphenyl group, a 4-(2-propenyl)phenyl group, a 3-(2-propenyl)phenyl group, a 2-(4-vinylphenyl)ethyl group, a 2-(3-vinylphenyl)ethyl group, a vinyl group, an allyl group, a methallyl group and a 5-hexenyl group.
R¹ is as defined previously,
Xⁱ represents a silylalkyl group that is represented by the following formula (IA), when i is equal to 1: 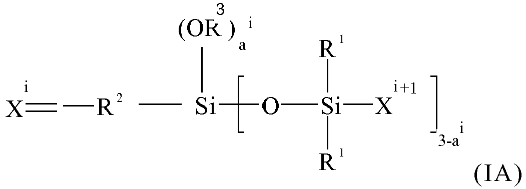 in which:
   R¹ is as defined above,
   R² represents an alkylene group containing from 2 to 10 carbon atoms, chosen from an ethylene group, a propylene group, a butylene group, a hexylene group or a similar linear alkylene group; a methylmethylene group, a methylethylene group, a 1-methylpentylene group, a 1,4-dimethylbutylene group or a similar branched alkylene group.
   R³ represents an alkyl group containing from 1 to 10 carbon atoms, chosen from methyl, ethyl, propyl, butyl and isopropyl groups,
   Xⁱ⁺¹ represents a hydrogen atom, an alkyl group containing from 1 to 10 carbon atoms, an aryl group or the silylalkyl group with i = i + 1,
   aⁱ is an integer ranging from 0 to 3 and i is an integer ranging from 1 to 10.

More preferentially, R² represents an alkylene group containing from 2 to 10 carbon atoms, chosen from ethylene, methylethylene, hexylene, 1-methylpentylene and 1,4-dimethylbutylene groups.

The superscript i in formula (IA) is an integer ranging from 1 to 10, which corresponds to the number of generations of said silylalkyl group, i.e. the number of times that the silylalkyl group is repeated.

For example, when the number of generations is equal to one, the carbosiloxanne dendrimer may be represented by general formula (III) as described hereinafter: 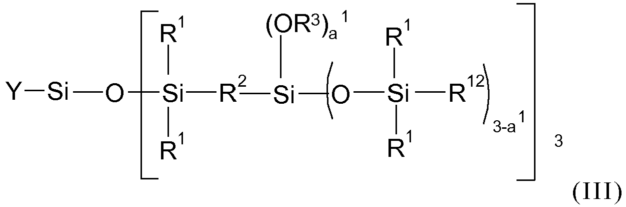 in which Y, R¹, R² and R³ are as defined above, R¹² represents a hydrogen atom or is identical to R¹; a¹ is an integer ranging from 0 to 3. Preferably, the total average number of groups OR³ in a molecule is within the range from 0 to 7.

When the number of generations is equal to 2, the carbosiloxane dendrimer is represented by general formula (IV) as described hereinafter: 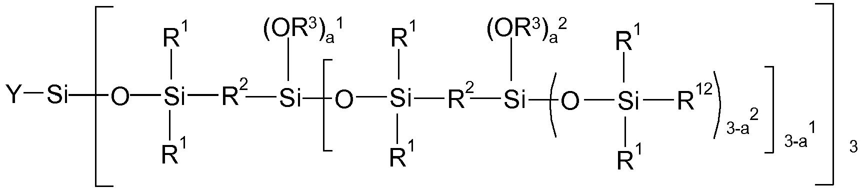 in which Y, R¹, R², R³ and R¹² are the same as defined above; a¹ and a² represent the aⁱ of the generation indicated. Preferably, the total average number of groups OR³ in a molecule is within the range from 0 to 25.

When the number of generations is equal to 3, the carbosiloxane dendrimer is represented by general formula (V) as described hereinafter:

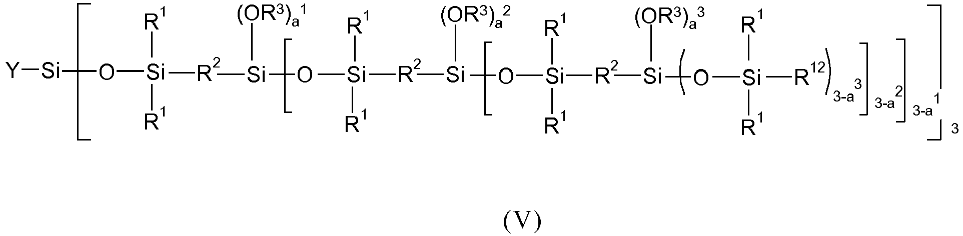

A carbosiloxane dendrimer that contains a radical-polymerizable organic group may be represented by the following average structural formulae:

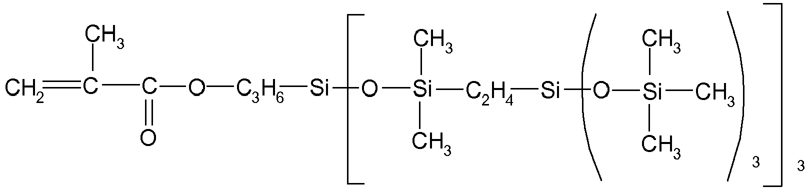

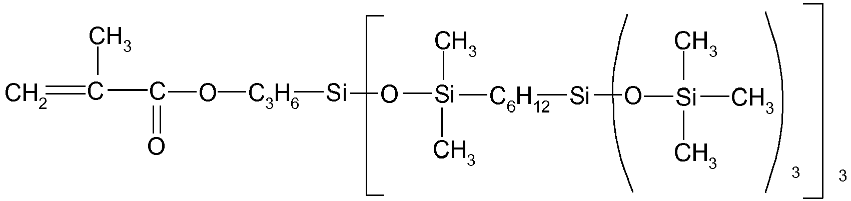

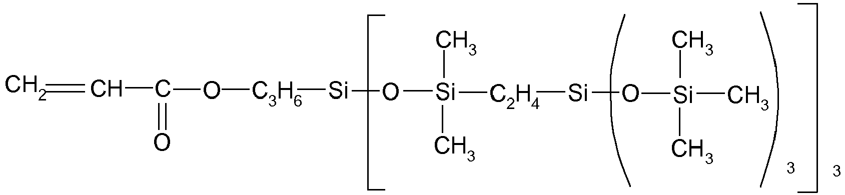

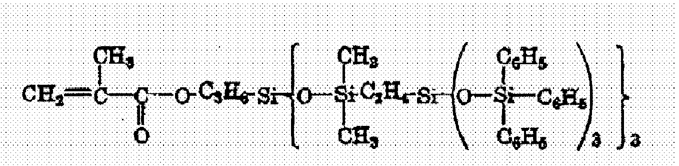

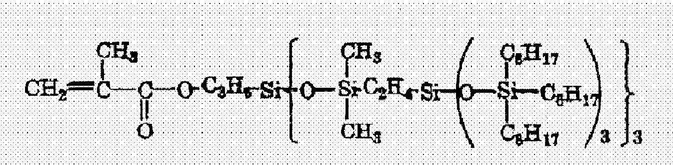

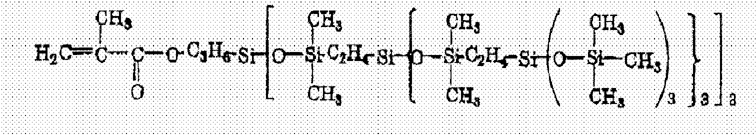

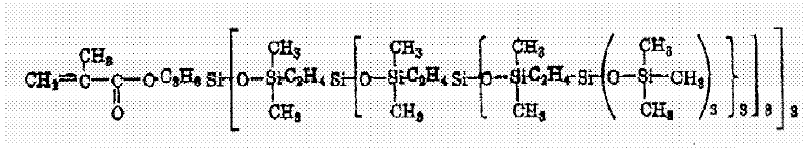

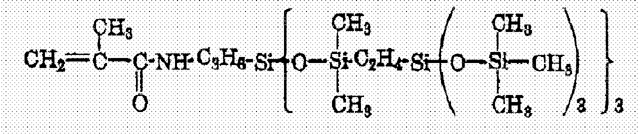

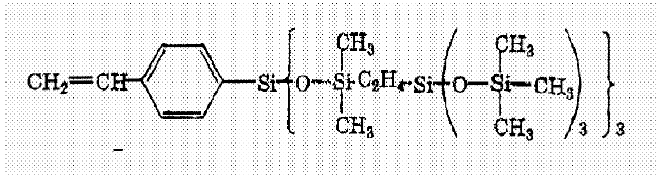

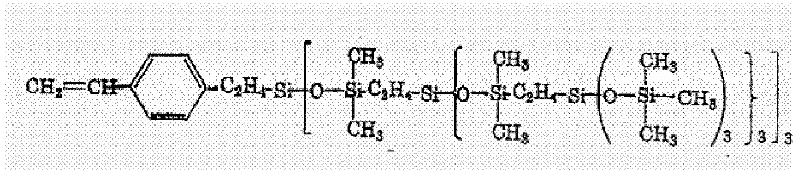

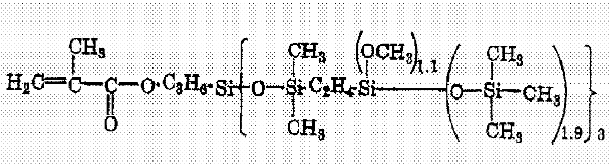

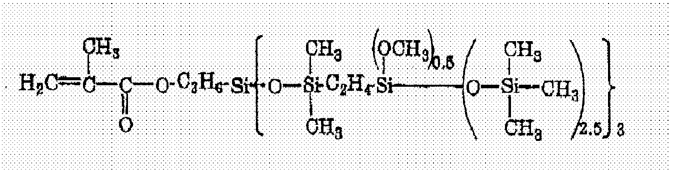

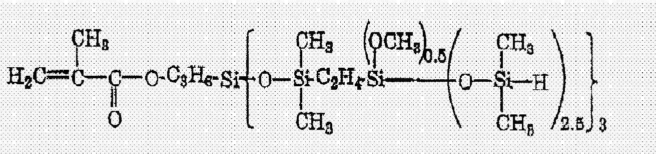

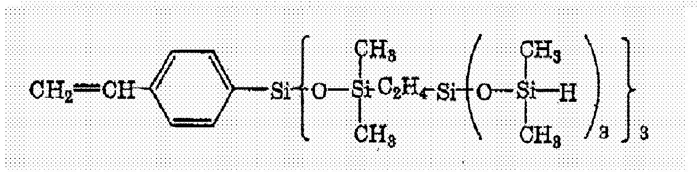

The carbosiloxane dendrimer may be manufactured according to the process for manufacturing a branched silalkylene siloxane described in Japanese patent application Hei 9-171 154.

For example, carbosiloxanne dendrimer may be produced by subjecting an organosilicon compound containing a hydrogen atom linked to a silicon atom, represented by the following general formula: 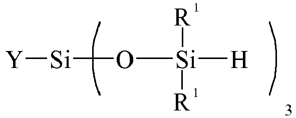 and an organosilicon compound containing an alkenyl group, to a hydrosilylation reaction.

In the above formula, the organosilicon compound may be represented by 3-methacryloxypropyltris(dimethylsiloxy)silane, 3-acryloxypropyltris(dimethylsiloxy)silane and 4-vinylphenyltris(dimethylsiloxy)silane.

The organosilicon compound that contains an alkenyl group may be represented by vinyltris(trimethylsiloxy)silane, vinyltris(dimethylphenylsiloxy)silane, and 5-hexenyltris(trimethylsiloxy)silane.

The hydrosilylation reaction is performed in the presence of a chloroplatinic acid, a complex of vinylsiloxane and of platinum, or a similar transition metal catalyst.

A vinyl polymer containing at least one carbosiloxane dendrimer-based unit may also be chosen from polymers containing a carbosiloxane dendrimer-based unit corresponding to a carbosiloxane dendritic structure represented by formula (VI): 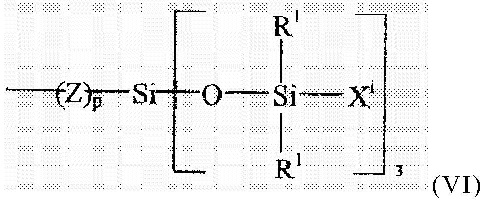 in which Z is a divalent organic group, p is 0 or 1, R¹ is an aryl or alkyl group of 1 to 10 carbon atoms and X' is a silylalkyl group which, when i = 1, is represented by the formula: 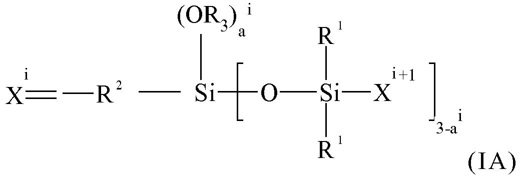 in which R¹ is as defined above, R² is an alkylene group containing from 1 to 10 carbon atoms, R³ is an alkyl group containing from 1 to 10 carbon atoms and Xi+1 is a group chosen from the group comprising hydrogen atoms, aryl groups and alkyl groups containing up to 10 carbon atoms, and silylalkyl groups Xi in which the superscript i is an integer ranging from 1 to 10 indicating the generation of the starting silylalkyl group in each carbosiloxane dendritic structure with a value of 1 for the group Xi in formula (IA) and the index ai is an integer ranging from 0 to 3.

In a vinyl polymer containing at least one carbosiloxane dendrimer-based unit, the polymerization ratio between the components (A) and (B), in terms of the weight ratio between (A) and (B), may be within a range from 0/100 to 99.9/0.1, or even from 0.1/99.9 to 99.9/0.1 and preferably within a range from 1/99 to 99/1. A ratio between the components (A) and (B) of 0/100 means that the compound becomes a homopolymer of component (B).

A vinyl polymer containing at least one carbosiloxane dendrimer-based unit may therefore be obtained by copolymerization of the components (A) and (B), or by polymerization of the component (B) alone.

The polymerization may be a free-radical polymerization or an ionic polymerization, but free-radical polymerization is preferred.

The polymerization may be performed by bringing about a reaction between the components (A) and (B) in a solution for a period of from 3 to 20 hours in the presence of a radical initiator at a temperature of from 50°C to 150°C.

A suitable solvent for this purpose is hexane, octane, decane, cyclohexane or a similar aliphatic hydrocarbon; benzene, toluene, xylene or a similar aromatic hydrocarbon; diethyl ether, dibutyl ether, tetrahydrofuran, dioxane or similar ethers; acetone, methyl ethyl ketone, methyl isobutyl ketone, diisobutyl ketone or similar ketones; methyl acetate, ethyl acetate, butyl acetate, isobutyl acetate or similar esters; methanol, ethanol, isopropanol, butanol or similar alcohols; octamethylcyclotetrasiloxane, decamethylcyclopentasiloxane, hexamethyldisiloxane, octamethyltrisiloxane or a similar organosiloxane oligomer.

A radical initiator may be any compound known in the art for standard free-radical polymerization reactions. The specific examples of such radical initiators are 2,2'-azobis(isobutyronitrile), 2,2'-azobis(2-methylbutyronitrile), 2,2'-azobis(2,4-dimethylvaleronitrile) or similar compounds of azobis type; benzoyl peroxide, lauroyl peroxide, tert-butyl peroxybenzoate, tert-butyl peroxy-2-ethylhexanoate or a similar organic peroxide. These radical initiators may be used alone or in a combination of two or more. The radical initiators may be used in an amount of from 0.1 to 5 parts by weight per 100 parts by weight of the components (A) and (B). A chain-transfer agent may be added. The chain-transfer agent may be 2-mercaptoethanol, butyl mercaptan, n-dodecyl mercaptan, 3-mercaptopropyltrimethoxysilane, a polydimethylsiloxane containing a mercaptopropyl group or a similar compound of mercapto type; methylene chloride, chloroform, carbon tetrachloride, 3-chloropropyltrimethoxysilane or a similar halogenated compound.

In the manufacture of the polymer of vinyl type, after the polymerization, the unreacted residual vinyl monomer may be removed under conditions of heating under vacuum.

To facilitate the preparation of the mixture of the starting material of cosmetic products, the number-average molecular weight of the vinyl polymer containing a carbosiloxane dendrimer may be chosen within the range between 3000 and 2 000 000 and preferably between 5000 and 800 000. It may be a liquid, a gum, a paste, a solid, a powder, or any other form The preferred forms are solutions consisting of the dilution of a dispersion or of a powder in solvents.

The vinyl polymer may be a dispersion of a polymer of vinyl type having a carbosiloxane dendrimer structure in its molecular side chain, in a liquid such as a silicone oil, an organic oil, an alcohol or water.

The silicone oil may be a dimethylpolysiloxane with the two molecular ends capped with trimethylsiloxy groups, a copolymer of methylphenylsiloxane and of dimethylsiloxane having the two molecular ends capped with trimethylsiloxy groups, a copolymer of methyl-3,3,3-trifluoropropylsiloxane and of dimethylsiloxane having the two molecular ends capped with trimethylsiloxy groups, or similar unreactive linear silicone oils, and also hexamethylcyclotrisiloxane, octamethylcyclotetrasiloxane, decamethylcyclopentasiloxane, dodecamethylcyclohexasiloxane or a similar cyclic compound. In addition to the unreactive silicone oils, modified polysiloxanes containing functional groups such as silanol groups, amino groups and polyether groups on the ends or within the molecular side chains may be used.

The organic oils may be isododecane, liquid paraffin, isoparaffin, hexyl laurate, isopropyl myristate, myristyl myristate, cetyl myristate, 2-octyldodecyl myristate; isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cocoa oil, jojoba oil, gum oil, sunflower oil, soybean oil, camellia oil, squalane, castor oil, cottonseed oil, coconut oil, egg yolk oil, polypropylene glycol monooleate, neopentyl glycol 2-ethylhexanoate or a similar glycol ester oil; triglyceryl isostearate, the triglyceride of a fatty acid of coconut oil, or a similar oil of a polyhydric alcohol ester; polyoxyethylene lauryl ether, polyoxypropylene cetyl ether or a similar polyoxyalkylene ether.

The alcohol may be any type that is suitable for use in combination with a cosmetic product starting material. For example, it may be ethanol, butanol, isopropanol or similar lower alcohols.

A solution or a dispersion of the alcohol should have a viscosity within the range from 10 to 10⁹ mPa.s at 25°C. To improve the sensory use properties in a cosmetic product, the viscosity should be within the range from 100 to 5 × 10⁸ mPa.s.

The solutions and dispersions may be readily prepared by mixing a vinyl polymer containing at least one carbosiloxane dendrimer-based unit with a silicone oil, an organic oil, an alcohol or water. The liquids may be present in the step of polymerization of a vinyl polymer containing at least one carbosiloxane dendrimer-based unit. In this case, the unreacted residual vinyl monomer should be completely removed by heat treatment of the solution or dispersion under atmospheric pressure or reduced pressure.

In the case of a dispersion, the dispersion of the polymer of vinyl type may be improved by adding a surfactant.

Such an agent may be hexylbenzenesulfonic acid, octylbenzenesulfonic acid, decylbenzenesulfonic acid, dodecylbenzenesulfonic acid, cetylbenzenesulfonic acid, myristylbenzenesulfonic acid or anionic surfactants of the sodium salts of these acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow-trimethylammonium hydroxide, coconut oil-trimethylammonium hydroxide, or a similar cationic surfactant; a polyoxyalkylene alkyl ether, a polyoxyalkylenealkylphenol, a polyoxyalkylene alkyl ester, the sorbitol ester of polyoxyalkylene, polyethylene glycol, polypropylene glycol, an ethylene oxide additive of diethylene glycol trimethylnonanol, and non-ionic surfactants of polyester type, and also mixtures.

In addition, the solvents and dispersions may be combined with iron oxide suitable for use with cosmetic products, or a similar pigment, and also zinc oxide, titanium oxide, silicon oxide, mica, talc or similar inorganic oxides in powder form. In the dispersion, a mean particle diameter of the polymer of vinyl type may be within a range of between 0.001 and 100 microns and preferably between 0.01 and 50 microns. The reason for this is that, outside the recommended range, a cosmetic product mixed with the emulsion will not have a nice enough feel on the skin or to the touch, nor sufficient spreading properties nor a pleasant feel.

A vinyl polymer contained in the dispersion or the solution may have a concentration within a range of between 0.1% and 95% by weight and preferably between 5% and 85% by weight. However, to facilitate the handling and the preparation of the mixture, the range should preferably be between 10% and 75% by weight.

A vinyl polymer that is suitable for use in the invention may also be one of the polymers described in the examples of patent application EP 0 963 751.

According to one preferred embodiment, a vinyl polymer grafted with a carbosiloxane dendrimer may be the product of polymerization of:
- from 0.1 to 99 parts by weight of one or more acrylate or methacrylate monomer(s); and
- from 100 to 0.1 parts by weight of an acrylate or methacrylate monomer of a tris[tri(trimethylsiloxy)silylethyldimethylsiloxy]silylpropyl carbosiloxane dendrimer.

According to one preferred embodiment, a vinyl polymer containing at least one carbosiloxane dendrimer-based unit may comprise a tris[tri(trimethylsiloxy)silylethyldimethylsiloxy]silylpropyl carbosiloxane dendrimer-based unit corresponding to one of the formulae: 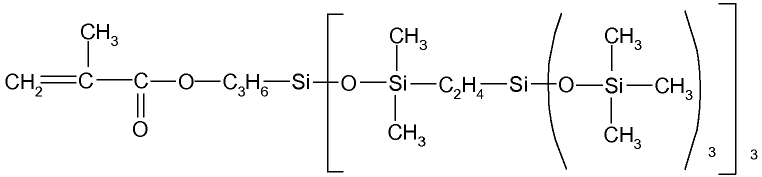 or

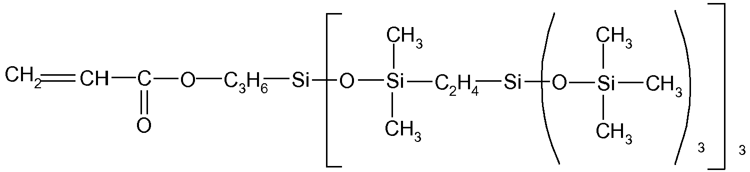

According to one preferred mode, a vinyl polymer containing at least one carbosiloxane dendrimer-based unit used in the invention comprises at least one butyl acrylate monomer. According to one embodiment, a vinyl polymer that can be used in the composition according to the invention may also comprise at least one fluoro organic group.

Structures in which the polymerized vinyl units constitute the backbone and carbosiloxane dendritic structures and also fluoro organic groups are attached to side chains are particularly preferred.

The fluoro organic groups may be obtained by replacing with fluorine atoms all or some of the hydrogen atoms of methyl, ethyl, propyl, isopropyl, butyl, isobutyl, tert-butyl, pentyl, neopentyl, hexyl, cyclohexyl, heptyl, octyl, nonyl, decyl, undecyl, dodecyl, tridecyl, tetradecyl, hexadecyl and octadecyl groups and other alkyl groups of 1 to 20 carbon atoms, and also alkyloxyalkylene groups of 6 to 22 carbon atoms.

The groups represented by the formula: -(CH₂)ₓ-(CF₂)_{y}-R¹³ are suggested as examples of fluoroalkyl groups obtained by substituting fluorine atoms for hydrogen atoms of alkyl groups. In the formula, the index "x" may correspond to 0, 1, 2 or 3, and "y" to an integer ranging from 1 to 20. R¹³ is an atom or a group chosen from a hydrogen atom, a fluorine atom, -(CH(CF₃)₂- or CF(CF₃)₂. Such fluorine-substituted alkyl groups are exemplified by linear or branched polyfluoroalkyl or perfluoroalkyl groups represented by the formulae shown hereinafter:
-CF₃, -C₂F₅, -nC₃F₇, -CF(CF₃)₂, -nC₄F₉, CF₂CF(CF₃)₂, -nC₅F₁₁, -nC₆F₁₃, -nC₈F₁₇, CH₂CF₃, -(CH(CF₃)₂, CH₂CH(CF₃)₂-CH₂(CF₂)₂F,-CH₂(CF₂)₃F, -CH₂(CF₂)₄F,CH₂(CF₂)₆F, CH₂(CF₂)₈F, -CH₂CH₂CF₃,-CH₂CH₂(CF₂)₂F, -CH₂CH₂(CF₂)₃F, -CH₂CH₂(CF₂)₄F, -CH₂CH₂(CF₂)₆F, -CH₂CH₂(CF₂)₈F, -CH₂CH₂(CF₂)₁₀F, -CH₂CH₂(CF₂)₁₂F, CH₂CH₂(CF₂)₁₄F, -CH₂CH₂(CF₂)₁₆F, -CH₂CH₂CH₂CF₃,-CH₂CH₂CH₂(CF₂)₂F, -CH₂CH₂CH₂(CF₂)₂H -CH₂(CF₂)₄H, and-CH₂CH₂(CF₂)₃H.

The groups represented by -CH₂CH₂-(CF₂)ₘ-CFR¹⁴-[OCF₂CF(CF₃)]ₙ-OC₃F₇ are suggested as fluoroalkyloxyfluoroalkylene groups obtained by substituting fluorine atoms for hydrogen atoms of alkyloxyalkylene groups.

In the formula, the index "m" is 0 or 1, "n" is 0, 1, 2, 3, 4 or 5, and R¹⁴ is a fluorine atom or CF₃. Such fluoroalkyloxyfluoroalkylene groups are illustrated by the perfluoroalkyloxyfluoroalkylene groups represented by the formulae presented hereinafter:
-CH₂CH₂CF(CF₃)-[OCF₂CF(CF₃)]n-OC₃F₇, -CH₂CH₂CF₂CF₂-[OCF₂CF(CF₃)]n-OC₃F₇.

The number-average molecular weight of the vinyl polymer used in the present invention may be between 3000 and 2 000 000 and more preferably between 5000 and 800 000.

In accordance with this embodiment, the vinyl polymer containing at least one carbosiloxane dendrimer-based unit may contain:
- one or more vinyl monomers (A) which do not comprise a fluoro organic group,
- one or more vinyl monomers containing one or more fluoro organic groups (C),
- one or more carbosiloxane dendrimer-based units (B) containing radical-polymerizable organic groups represented by general formula (I): 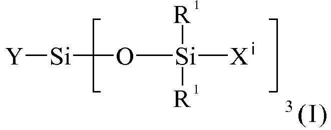 in which Y, R¹ and Xⁱ or as defined above.

Thus, the vinyl polymer containing at least one carbosiloxane dendrimer-based unit can be obtained by addition:
- of one or more vinyl monomers (A) which do not comprise a fluoro organic group,
- on one or more vinyl monomers containing one or more fluoro organic groups (C), and
- one or more carbosiloxane dendrimers (B) containing radical-polymerizable organic groups represented by general formula (I): 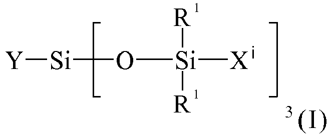 in which Y is a radical-polymerizable organic group and R¹ and Xⁱ are as defined above, and by subjecting them to a copolymerization.

Thus, according to one embodiment, a composition according to the invention may comprise a vinyl polymer containing at least one carbosiloxane dendrimer-based unit which is the product of copolymerization:
- of one or more vinyl monomers containing fluoro organic groups (C),
- optionally of one or more vinyl monomers not containing a fluoro organic group (A), and
- of one or more carbosiloxane dendrimers (B) containing radical-polymerizable organic groups represented by general formula (I): 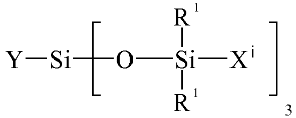 in which Y is a radical-polymerizable organic group, R¹ is an aryl or alkyl group of 1 to 10 carbon atoms and X' is a silylalkyl group represented by the following formula (IA): 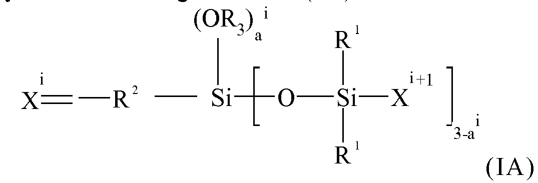 in which R¹ is as above, R² is an alkylene group of 2 to 10 carbon atoms, R³ is an alkyl group of 1 to 10carbon atoms, and Xⁱ⁺¹ is a group chosen from the group comprising hydrogen atoms, aryl groups and alkyl groups containing up to 10 carbon atoms, and silylalkyl groups Xⁱ mentioned above in which the superscript i is an integer ranging from 1 to 10 indicating the generation of said starting silylalkyl group in each carbosiloxane dendritic structure with a value of 1 for the group Xⁱ in formula (I), the index aⁱ is an integer ranging from 0 to 3, said vinyl polymer having a copolymerization ratio of the component (C) to the component (B) of 0.1 to 100:99.9 to 0% by weight, and a copolymerization ratio of the sum of the component (C) and of the component (A) to the component (B) of 0.1 to 99.9:99.9 to 0.1% by weight.

The vinyl monomers containing fluoro organic groups (C) are preferably monomers represented by the general formula:

(CH₂)=CR¹⁵COOR_{f}

In the formula, R¹⁵ is a hydrogen atom or a methyl group and Rf is an fluoro organic group exemplified by the fluoroalkyl and fluoroalkyloxyfluoroalkylene groups described above. The compounds represented by the formulae presented below are suggested as specific examples of the component (C). In the formulae presented below, z is an integer ranging from 1 to 4.
CH₂=CCH₃COO-CF₃, CH₂=CCH₃COO-C₂F₅,
CH₂=CCH₃COO-nC₃F₇,
CH₂=CCH₃COO-CF(CF₃)₂, CH₂=CCH₃COO-nC₄F₉,
CH₂=CCH₃COO-CF(CF₃)₂, CH₂=CCH₃COO-nC₅F₁₁,
CH₂=CCH₃COO-nC₆F₁₃,
CH₂=CCH₃COO-nC₈F₁₇, CH₂=CCH₃COO-CH₂CF₃,
CH₂=CCH₃COO-CH(CF₃)₂, CH₂=CCH₃COO-CH₂CH(CF₃)₂,
CH₂=CCH₃COO-CH₂(CF₂)₂F,
CH₂=CCH₃COO-CH₂(CF₂)₂F, CH₂=CCH₃COO-CH₂(CF₂)₄F,
CH₂=CCH₃COO-CH₂(CF₂)₆F, CH₂=CCH₃COO-CH₂(CF₂)₈F,
CH₂=CCH₃COO-CH₂CH₂CF₃,
CH₂=CCH₃COO-CH₂CH₂(CF₂)₂F,
CH₂=CCH₃COO-CH₂CH₂(CF₂)₃F,
CH₂=CCH₃COO-CH₂CH₂(CF₂)₄F,
CH₂=CCH₃COO-CH₂CH₂(CF₂)₆F,
CH₂=CCH₃COO-CH₂CH₂(CF₂)₈F,
CH₂=CCH₃COO-CH₂CH₂(CF₂)₁₀F,
CH₂=CCH₃COO-CH₂CH₂(CF₂)₁₂F,
CH₂=CCH₃COO-CH₂CH₂(CF₂)₁₄F,
CH₂=CCH₃COO-CH₂-CH₂-(CF₂)₁₆F,
CH₂=CCH₃COO-CH₂CH₂ CH₂CF₃,
CH₂=CCH₃COO-CH₂CH₂CH₂(CF₂)₂F,
CH₂=CCH₃COO-CH₂CH₂CH₂(CF₂)₂H, CH₂=CCH₃COO-CH₂ (CF₂)₄H,
CH₂=CCH₃COO-(CF₂)₃H,
CH₂=CCH₃COO-CH₂CH₂CF(CF₃)-[OCF₂-CF(CF₃)]_{z}-OC₃F₇,
CH₂=CCH₃COO-CH₂CH₂CF₂CF₂-[OCF₂-CF(CF₃)]_{z}-OC₃F₇,
CH₂=CHCOO-CF₃, CH₂=CHCOO-C₂F₅, CH2=CHCOO-nC₃F₇, CH₂=CHCOO-CF(CF₃)₂,
CH₂=CHCOO-nC₄F₉, CH₂=CHCOO-CF₂CF(CF₃)₂, CH2=CHCOO-nC₅F₁₁,
CH₂=CHCOO-nC₆F₁₃, CH₂=CHCOO-nC₈F₁₇, CH₂=CHCOO-CH₂CF₃,
CH₂=CHCOO-CH(CF₃)₂, CH₂=CHCOO-CH₂CH(CF₃)₂, CH₂=CHCOO-CH₂(CF₂)₂F,
CH₂=CHCOO-CH₂(CF₂)₃F, CH₂=CHCOO-CH₂(CF₂)₄F, CH₂=CHCOO-CH₂(CF₂)₆F,
CH₂=CHCOO-CH₂(CF₂)₈F, CH₂=CHCOO-CH₂CH₂CF₃,
CH₂=CHCOO-CH₂CH₂(CF₂)₂F,
CH₂=CHCOO-CH₂CH₂(CF₂)₃F, CH₂=CHCOO-CH₂CH₂(CF₂)₄F,
CH₂=CHCOO-CH₂CH₂(CF₂)₆F, CH₂=CHCOO-CH₂CH₂(CF₂)₈F,
CH₂=HCOO-CH₂CH₂(CF₂)₁₀F, CH₂-CHCOO-CH₂CH₂-(CF₂)₁₂F,
CH₂=CHCOO-CH₂CH₂(CF₂)₁₄F, CH₂=CHCOO-CH₂CH₂(CF₂)₁₆F,
CH₂=CHCOO-CH₂CH₂CH₂CF₃, CH₂=CHCOO-CH₂CH₂CH₂(CF₂)₂F,
CH₂=CHCOO-CH₂CH₂CH₂(CF)₂H, CH₂=CHCOO-CH₂(CF₂)₄H,
CH₂=CHCOO-CH₂CH₂(CF₂)₃H,
CH₂=CHCOO-CH₂CH₂CF(CF₃)-[OCF₂-CF(CF₃)]_{z}-OC₃F₇,
CH₂=CHCOO-CH₂CH₂CF₂CF₂(CF₃)-[OCF₂-CF(CF₃)]₂-OC₃F₇.

Preferably, the vinyl polymers contain one or more vinyl monomers containing fluoro groups having the formulae:

CH₂=CHCOO-CH₂CH₂(CF₂)₆F, CH₂=CHCOO-CH₂CH₂(CF₂)₈F,

CH₂=CCH₃COO-CH₂CH₂(CF₂)₆F, CH₂=CCH₃COO-CH₂CH₂(CF₂)₈F,

CH₂=CHCOO-CH₂CF₃, CH₂=CCH₃COO-CH₂CF₃.

More preferentially, the vinyl polymers contain one or more vinyl monomers containing fluoro groups having the formula:

CH₂=CHCOO-CH₂CF₃, CH₂=CCHCOO-CH₂CF₃.

The vinyl monomers (A) not containing any organofluorine groups in the molecule may be monomers containing radical-polymerizable vinyl groups which are illustrated, for example, by methyl acrylate, methyl methacrylate, ethyl acrylate, ethyl methacrylate, n-propyl acrylate, n-propyl methacrylate, isopropyl acrylate, isopropyl methacrylate, and other lower alkyl acrylates or methacrylates; glycidyl acrylate, glycidyl methacrylate; n-butyl acrylate, n-butyl methacrylate, isobutyl acrylate, isobutyl methacrylate, tert-butyl acrylate, tert-butyl methacrylate, n-hexyl acrylate, n-hexyl methacrylate, cyclohexyl acrylate, cyclohexyl methacrylate, 2-ethylhexyl acrylate, 2-ethylhexyl methacrylate, octyl acrylate, octyl methacrylate, lauryl acrylate, lauryl methacrylate, stearyl acrylate, stearyl methacrylate, and other higher acrylates and methacrylates; vinyl acetate, vinyl propionate and other lower fatty acid vinyl esters; vinyl butyrate, vinyl caproate, vinyl 2-ethylhexanoate, vinyl laurate, vinyl stearate, and other higher fatty acid esters; styrene, vinyltoluene, benzyl acrylate, benzyl methacrylate, phenoxyethyl acrylate, phenoxyethyl methacrylate, vinylpyrrolidone, and other vinyl aromatic monomers; dimethylaminoethyl acrylate, dimethylaminoethyl methacrylate, diethylaminoethyl acrylate, diethylaminoethyl methacrylate, and other aminovinyl monomers, acrylamide, methacrylamide, N-methylolacrylamide, N-methylolmethacrylamide, N-methoxymethylacrylamide, N-methoxymethylmethacrylamide, isobutoxymethoxyacrylamide, isobutoxymethoxymethacrylamide, N,N-dimethylacrylamide, N,N-dimethylmethacrylamide, and other vinylamide monomers; hydroxyethyl acrylate, hydroxyethyl methacrylate, acrylic acid hydroxypropyl alcohol, methacrylic acid hydroxypropyl alcohol, and other hydroxyvinyl monomers; acrylic acid, methacrylic acid, itaconic acid, crotonic acid, fumaric acid, maleic acid, and other vinylcarboxylic acid monomers; tetrahydrofurfuryl acrylate, tetrahydrofurfuryl methacrylate, butoxyethyl acrylate, butoxyethyl methacrylate, ethoxydiethylene glycol acrylate, ethoxydiethylene glycol methacrylate, polyethylene glycol acrylate, polyethylene glycol methacrylate, polypropylene glycol monoacrylate, polypropylene glycol monomethacrylate, hydroxybutyl vinyl ether, cetyl vinyl ether, 2-ethylhexyl vinyl ether, and other vinyl monomers containing an ether bond; acryloxypropyltrimethoxysilane, methacryloxypropyltrimethoxysilane, polydimethylsiloxanes containing acryl or methacryl groups at one of the ends, polydimethylsiloxanes containing alkenylaryl groups at one of the ends and other silicone compounds containing unsaturated groups; butadiene; vinyl chloride; vinylidene chloride, acrylonitrile, methacrylonitrile; dibutyl fumarate; maleic anhydride; dodecylsuccinic anhydride; acryl glycidyl ether, methacryl glycidyl ether, 3,4-epoxycyclohexylmethyl acrylate, 3,4-epoxycyclohexylmethyl methacrylate, alkali metal salts, ammonium salts and organic amine salts of acrylic acid, of methacrylic acid, of itaconic acid, of crotonic acid, of fumaric acid, of maleic acid and of other radical-polymerizable unsaturated carboxylic acids, radical-polymerizable unsaturated monomers containing sulfonic acid groups, such as styrene sulfonic acid and also the alkali metal salts thereof, the ammonium salts thereof and the organic amine salts thereof; the quaternary ammonium salts derived from acrylic acid or methacrylic acid, such as 2-hydroxy-3-methacryloxypropyltrimethyl ammonium chloride, methacrylic acid esters of a tertiary amine alcohol, such as the diethylamine ester of methacrylic acid and quaternary ammonium salts thereof.

In addition, it is also possible to use as vinyl monomers (A) the polyfunctional vinyl monomers illustrated, for example, by trimethylolpropane triacrylate, trimethylolpropane trimethacrylate, pentaerythrityl triacrylate, pentaerythrityl trimethacrylate, ethylene glycol diacrylate, ethylene glycol dimethacrylate, tetraethylene glycol diacrylate, tetraethylene glycol dimethacrylate, polyethylene glycol diacrylate, polyethylene glycol dimethacrylate, 1,4-butanediol diacrylate, 1,4-butanediol dimethacrylate, 1,6-hexanediol diacrylate, 1,6-hexanediol dimethacrylate, neopentyl glycol diacrylate, neopentyl glycol dimethacrylate, trimethylolpropanetrioxyethyl acrylate, trimethylolpropanetrioxyethyl methacrylate, tris(2-hydroxyethyl)isocyanurate diacrylate, tris(2-hydroxyethyl)isocyanurate dimethacrylate, tris(2-hydroxyethyl)isocyanurate triacrylate, tris(2-hydroxyethyl)isocyanurate trimethacrylate, polydimethylsiloxane in which the two ends of the molecular chain are blocked with alkenylaryl groups, and other silicone compounds containing unsaturated groups.

As regards the ratio mentioned above in which the component (C) and the component (A) are copolymerized, the weight ratio of compound (C) to compound (A) should be within the range from 0.1:99.9 to 100:0 and preferably within the range 1:99 to 100:0.

In the component (B), Y a radical-polymerizable organic group which can be chosen from organic groups containing acrylic or methacrylic groups, organic groups containing an alkenylaryl group, or alkenyl groups containing from 2 to 10 carbon atoms.

The organic groups containing acrylic or methacrylic groups may be represented by the general formulae: 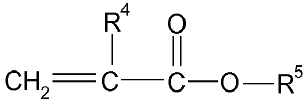 and

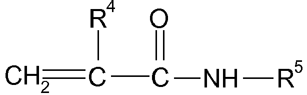

The alkenylaryl group may be represented by the formula:

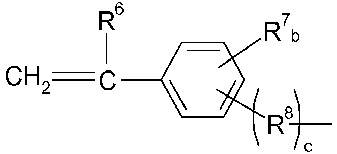

In the formulae above, R⁴ and R⁶ are hydrogen atoms or methyl groups, R⁵ and R⁸ are alkylene groups of 1 to 10 carbon atoms, and R⁷ is an alkyl group of 1 to 10 carbon atoms. The index "b" is an integer from 0 to 4, and "c" is 0 or 1.

Preferably, Y is a radical-polymerizable organic group which can be chosen from acryloxymethyl, 3-acryloxypropyl, methacryloxymethyl, 3-methacryloxypropyl, 4-vinylphenyl, 3-vinylphenyl, 4-(2-propenyl)phenyl, 3-(2-propenyl)phenyl, 2-(4-vinylphenyl)ethyl, 2-(3-vinylphenyl)ethyl, vinyl, allyl, methallyl and 5-hexenyl.

In accordance with this embodiment, the first-generation carbosiloxane dendrimer may be represented by formula (IV), the second-generation carbosiloxane dendrimer may be represented by formula (V), and the third-generation carbosiloxane dendrimer may be represented by formula (VI).

In accordance with this embodiment, the carbosiloxane dendrimer (B) may be chosen from formulae (VI) to (XIX) as described above.

In particular, the carbosiloxane dendrimer-based unit comprises at least one tris[tri(trimethylsiloxy)silylethyldimethylsiloxy]silylpropyl carbosiloxane dendrimer-based unit corresponding to one of the formulae:

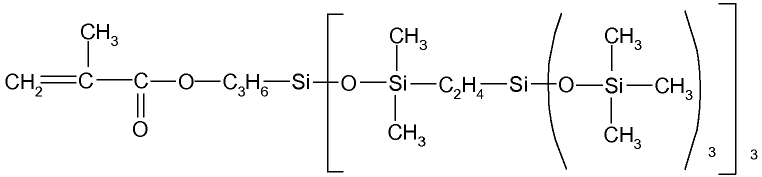

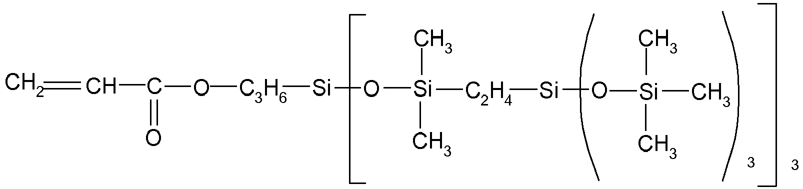

The carbosiloxane dendrimers of the component (C) may be prepared using the process for preparing siloxane/silalkylene branched copolymers described in document EP 1 055 674.

For example, they may be prepared by subjecting organic alkenyl silicone compounds and silicone compounds comprising hydrogen atoms linked to silicon, represented by the general formula: 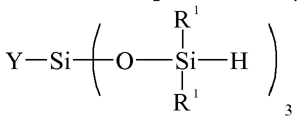 in which R¹ and Y are as defined above, to a hydrosilylation reaction.

The copolymerization ratio of the component (B), in terms of its weight ratio relative to the total of compound (C) and of compound (A) may be within the range from 0.1:99.9 to 99.9:0.1, preferably within the range from 1:99 to 99:1 and even more preferably within the range from 5:95 to 95:5.

Amino groups may be introduced into the side chains of the vinyl polymer using, included in the component (A), vinyl monomers containing amino groups, such as dimethylaminoethyl acrylate, dimethylaminoethyl methacrylate, diethylaminoethyl acrylate and diethylaminoethyl methacrylate, followed by performing a modification with potassium acetate monochloride, ammonium acetate monochloride, the aminomethylpropanol salt of monochloroacetic acid, the triethanolamine salt of monobromoacetic acid, sodium monochloropropionate, and other alkali metal salts of halogenated fatty acids; otherwise, carboxylic acid groups may be introduced into the side chains of the vinyl polymer using, included in the component (B), vinyl monomers containing carboxylic acids, such as acrylic acid, methacrylic acid, itaconic acid, crotonic acid, fumaric acid and maleic acid, and the like, followed by neutralizing the product with triethylamine, diethylamine, triethanolamine and other amines.

A fluoro vinyl polymer may be one of the polymers described in the examples of patent application WO 03/045 337.

According to one preferred embodiment, a vinyl polymer grafted in the sense of the present invention may be conveyed in an oil or a mixture of oils, which is/are preferably volatile, chosen in particular from silicone oils and hydrocarbon-based oils, and mixtures thereof.

According to one particular embodiment, a silicone oil that is suitable for use in the invention may be cyclopentasiloxane.

According to another particular embodiment, a hydrocarbon-based oil that is suitable for use in the invention may be isododecane.

Preferably, the vinyl polymer containing at least one carbosiloxane dendrimer-based unit does not contain a vinyl monomer-based unit containing fluoro organic groups.

The vinyl polymers grafted with at least one carbosiloxane dendrimer-based unit that may be particularly suitable for use in the present invention are the polymers known under the INCI name Acrylates/Polytrimethylsiloxymethacrylate and sold under the names TIB 4-100, TIB 4-101, TIB 4-120, TIB 4-130, TIB 4-200, FA 4002 ID (TIB 4-202), TIB 4-220 and FA 4001 CM (TIB 4-230) by the company Dow Corning.

The vinyl polymers grafted with at least one carbosiloxane dendrimer-based unit are preferably present in the compositions of the invention in proportions ranging from 0.01% to 20% by weight relative to the total weight of the composition, preferably ranging from 0.1% to 10% by weight relative to the total weight of the composition, and even more preferentially from 0.5% to 5% by weight relative to the total weight of the composition.

### Olefin copolymer

The olefin copolymer in accordance with the invention is preferably chosen from amorphous olefin copolymers or olefin copolymers with controlled and moderate crystallization.

For the purpose of the present patent application, the term "olefin copolymer" is intended to mean any copolymer formed by polymerization of at least one olefin and of another additional monomer other than said olefin.

The olefin may in particular be an ethylenically unsaturated monomer.

Examples of olefins that may be mentioned include ethylenic carbide monomers in particular containing one or two ethylenic unsaturations, containing from 2 to 5 carbon atoms, such as ethylene, propylene, butadiene or isoprene.

### Amorphous olefin copolymer

According to a first embodiment, the olefin copolymer may be an amorphous copolymer formed by polymerization of at least one olefin.

The term "amorphous copolymer" is intended to mean a polymer that does not have a crystalline form. The amorphous copolymer is also film-forming, i.e. it is capable of forming a film when applied to the skin.

The amorphous olefin copolymer may in particular be a diblock, triblock, multiblock, radial or star copolymer, or mixtures thereof.

Such amorphous olefin copolymers are described in patent application US-A-2002/005562 and in patent US-A-5 221 534.

Advantageously, the amorphous olefin copolymer is an amorphous block copolymer of styrene and of an olefin.

The amorphous olefin copolymer is preferably hydrogenated to reduce the residual ethylenic unsaturations after polymerization of the monomers.

In particular, the amorphous olefin copolymer is an optionally hydrogenated copolymer, containing styrene blocks and ethylene/C₃-C₄ alkylene blocks.

Diblock copolymers, which are preferably hydrogenated, that may be mentioned include styrene/ethylene-propylene copolymers and styrene/ethylene-butadiene copolymers. Diblock polymers are in particular sold under the name Kraton® G1701E by the company Kraton Polymers.

Triblock copolymers, which are preferably hydrogenated, that may be mentioned include styrene/ethylene-propylene/styrene copolymers, styrene/ethylene-butadiene/styrene copolymers, styrene/isoprene/styrene copolymers and styrene/butadiene/styrene copolymers. Triblock polymers are in particular sold under the names Kraton® G1650, Kraton® G1652, Kraton® D1101, Kraton® D1102 and Kraton® D1160 by the company Kraton Polymers.

A mixture of hydrogenated styrene/butylene-ethylene/styrene triblock copolymer and of hydrogenated ethylene/propylene/styrene star polymer may also be used, such a mixture in particular being in isododecane. Such mixtures are sold, for example, by the company Penreco under the trade names Versagel® M5960 and Versagel® M5670.

Advantageously, a diblock copolymer such as those described previously, in particular a styrene/ethylene-propylene diblock copolymer bearing the INCI name Hydrogented Styrene/Isoprene Copolymer, is used as an amorphous olefin copolymer.

### Olefin copolymer with controlled and moderate crystallization

According to a second embodiment, the olefin copolymer is an olefin copolymer with controlled and moderate crystallization.

The olefin copolymers with controlled and moderate crystallization which are used in the composition of the present patent application may be any olefin copolymer, namely a copolymer containing only olefin units, having a controlled and moderate crystalline nature, i.e. a degree of crystallinity at most equal to 50%, preferably ranging from 5% to 40% and better still ranging from 10% to 35%.

These copolymers are generally elastomers or plastomers and may be synthesized via any known process, in particular via a radical route, via Ziegler-Natta catalysis or via metallocene catalysis. Such polymers are described in particular in patent application EP-A-1 034 776.

A first class of olefin copolymers with controlled and moderate crystallization that may be used in the compositions according to the invention may be linear or branched α-olefin copolymers, in particular C₂-C₁₆ and better still C₂-C₁₂ α-olefin copolymers. Preferably, these copolymers are bipolymers or terpolymers and most particularly bipolymers.

Among the bipolymers that are recommended for the compositions of the invention, mention may be made of bipolymers of ethylene and of a C₄-C₁₆ and preferably C₄-C₁₂ α-olefin and bipolymers of propylene and of a C₄-C₁₆ and preferably C₄-C₁₂ α-olefin. More preferably, the α-olefin is chosen from 1-butene, 1-pentene, 1-hexene, 1-octene, 1-nonene, 1-decene, 1-undecene, 1-dodecene, 3,5,5-trimethyl-1-hexene, 3-methyl-1-pentene and 4-methyl-1-pentene.

Among these monomers, 1-butene and 1-octene are particularly preferred.

The recommended bipolymers are elastomers which have a degree of crystallinity ranging from 10% to 35%.

These bipolymers are preferentially synthesized via metallocene catalysis.

Such bipolymers are sold by the company Dow Chemical under the trade name Affinity (plastomers) and by the company Dupont de Nemours under the name Engage (elastomers).

Ethylene/butene bipolymers are sold by the company Exxon under the trade name Exact Resins and by the company Elenac under the trade name Luflexen.

Among the terpolymers, mention may be made of terpolymers of ethylene, propylene and a C₄-C₁₆ and preferably C₄-C₁₂ α-olefin.

In these terpolymers, the contents of C₄-C₁₆ α-olefin are as indicated previously and the preferred α-olefins are butene, hexene and octene.

The preferred copolymers, described in the patent application EP-A-1 034 776, may in particular be ethylene/octene copolymers sold under the reference Engage 8400 by the company Dupont de Nemours.

A second class of olefin copolymers with controlled and moderate crystallization that are suitable for use in the invention are copolymers of ethylene or of propylene and of a cycloolefin, in particular bipolymers.

Generally, the cycloolefin content of the copolymers is less than 20 mol%.

Among the cycloolefins that may be used, mention may be made of cyclobutene, cyclohexene, cyclooctadiene, norbornene, dimethanooctahydronaphthalene (DMON), ethylidenenorbornene, vinylnorbornene and 4-vinylcyclohexene.

The recommended copolymers of this class are copolymers of ethylene and of norbornene. The norbornene content of these copolymers is generally less than 18 mol% to have the required crystalline nature, and these copolymers are synthesized via metallocene catalysis.

Suitable ethylene/norbornene copolymers are sold by the companies Mitsui Petrochemical or Mitsui-Sekka under the trade name Appel and by the company Hoechst-Celanese under the trade name Topas.

Other recommended ethylene/cycloolefin copolymers are ethylene/cyclobutene and ethylene/cyclohexene bipolymers with a low content of cycloolefin, generally less than 20 mol%.

A third class of olefin copolymers that are suitable for the present invention consists of copolymers of a monoolefin and of a monomer comprising a ethylenic bond(s), such as dienes, for example ethylene/butadiene, propylene/butadiene, ethylene/isoprene and propylene/isoprene bipolymers, and ethylene/propylene/diene terpolymers, also obtained via metallocene synthesis.

The proportion of "ethylene" or "diene" units in the copolymer with controlled crystallization is generally included in the range of from 3 mol% to 20 mol%.

According to one preferred embodiment, the olefin copolymer with controlled and moderate crystallization is chosen from ethylene/octene copolymers and ethylene/norbornene copolymers.

According to one preferred embodiment, the olefin copolymer may in particular be a polymeric gelling agent capable of thickening or gelling the organic phase of the composition.

According to one preferred embodiment, the olefin copolymer may in particular be film-forming.

For the purpose of the present patent application, the term "film-forming polymer" is intended to mean a polymer capable, by itself or using an auxiliary film-forming agent, of forming a macroscopically continuous film on a support, in particular on keratin materials, preferably a cohesive film and even better still a film of which the cohesion and mechanical properties are such that said film can be isolated from said support.

According to one preferred embodiment, the olefin copolymer is chosen from amorphous olefin copolymers, in particular from diblock copolymers such as those of styrene/ethylene-propylene described previously.

The olefin copolymer may be present in the composition according to the invention in a content ranging from 0.1% to 10% by weight, relative to the total weight of the composition, preferably ranging from 0.2% to 5% by weight and preferentially ranging from 0.5% to 3% by weight.

Preferably, the antiperspirant cosmetic composition comprises
(i) one or more vinyl polymers containing at least one carbosiloxane dendrimer-based unit comprising at least one tris[tri(trimethylsiloxy)silylethyldimethylsiloxy]silylpropyl carbosiloxane dendrimer-based unit corresponding to one of the formulae: 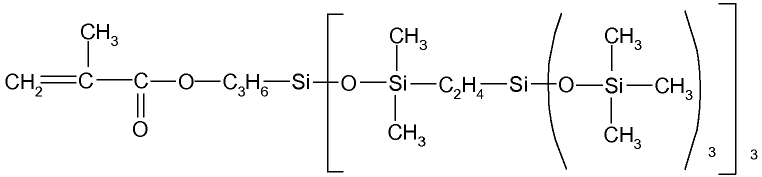 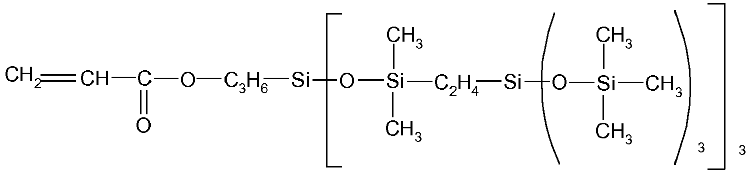
(ii) one or more diblock amorphous olefin copolymers such as those of styrene/ethylene-propylene.

For the purpose of the present invention, the term "cosmetically acceptable medium" is intended to mean a medium that is suitable for the topical administration of a composition. A physiologically acceptable medium is preferably a cosmetically or dermatologically acceptable medium, that is to say a medium which is devoid of unpleasant odour or appearance and which is entirely compatible with the topical administration route. In the present case, where the composition is intended for topical administration, that is to say for administration by application at the surface of the keratin material under consideration, such a medium is considered in particular to be physiologically acceptable when it does not cause stinging, tightness or redness unacceptable to the user.

The term "water-in-oil emulsion" is intended to mean a composition comprising a continuous oily phase and an aqueous phase dispersed in the oily phase; the two phases being stabilized by an emulsifying system, a thickening system or fillers.

In other words, the cosmetic composition according to the invention is an emulsion which can contain an aqueous phase and an oily phase.

### Aqueous phase

For the purpose of the present invention, the term "aqueous phase" is intended to mean the water and all the ingredients of the composition according to the invention which are soluble in water.

The aqueous phase preferably ranges from 50% to 80% by weight relative to the total weight of the composition.

The water represents an amount preferably ranging from 30% to 60% by weight and more preferentially from 30% to 50%, by weight relative to the total weight of the composition.

The aqueous phase may contain solvents other than water, such as polyols, for instance butylene glycol, hexanediol, glycerol, 1,3-propanediol, propylene glycol or ethanol.

### Water-in-oil emulsifier

The cosmetic composition according to the invention may comprise one or more emulsifiers chosen from silicone emulsifiers of the alkyl dimethicone copolyol type and of the dimethicone copolyol type, non-silicone W/O emulsifiers with an HLB of 3 to 7, and mixtures thereof.

### Emulsifiers of the alkyl dimethicone copolyol type and of the dimethicone copolyol type

The alkyl dimethicone copolyols in accordance with the invention correspond to the following formula (I): 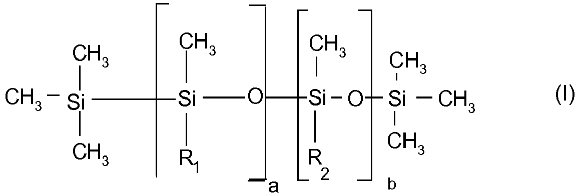 in which:
R₁ denotes a linear or branched C₁₂-C₂₀ and preferably C₁₂-C₁₈ alkyl group;
R₂ denotes the group: --CₙH₂ₙ--(-OC₂H₄-)ₓ--(-OC₃H₆-)_{y}--O-R₃;
R₃ denotes a hydrogen atom or a linear or branched alkyl radical containing from 1 to 12 carbon atoms;
a is an integer ranging from 1 to approximately 500;
b denotes an integer ranging from 1 to approximately 500;
n is an integer ranging from 2 to 12 and preferably from 2 to 5;
x denotes an integer ranging from 1 to approximately 50 and preferably from 1 to 30;
y denotes an integer ranging from 0 to approximately 49 and preferably from 0 to 29, with the proviso that, when y is other than zero, the ratio x/y is greater than 1 and preferably ranges from 2 to 11.

Among the alkyl dimethicone copolyol emulsifiers of formula (I) that are preferred, mention will be made more particularly of Cetyl PEG/PPG-10/1 Dimethicone and more particularly the mixture Cetyl PEG/PPG-10/1 Dimethicone and Dimethicone (INCI name), for instance the product sold under the trade name Abil EM90 by the company Goldschmidt, or alternatively the mixture (Polyglyceryl-4 Stearate and Cetyl PEG/PPG-10 (and) Dimethicone (and) Hexyl Laurate), for instance the product sold under the trade name Abil WE09 by the same company.

The dimethicone copolyols in accordance with the invention correspond to the following formula (II): 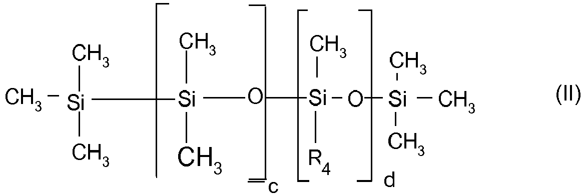 in which:
R₄ denotes the group: --CₘH₂ₘ--(-OC₂H₄-)ₛ--(-OC₃H₆-)ₜ--O-R₅,
R₅ denotes a hydrogen atom or a linear or branched alkyl radical comprising from 1 to 12 carbon atoms;
c is an integer ranging from 1 to approximately 500;
d denotes an integer ranging from 1 to approximately 500;
m is an integer ranging from 2 to 12 and preferably from 2 to 5;
s denotes an integer ranging from 1 to approximately 50 and preferably from 1 to 30;
t denotes an integer ranging from 0 to approximately 50 and preferably from 0 to 30; with the proviso that the sum s+t is greater than or equal to 1.

Among these preferential dimethicone copolyol emulsifiers of formula (II), use will particularly be made of PEG-18/PPG-18 Dimethicone and more particularly the mixture Cyclopentasiloxane (and) PEG-18/PPG-18 Dimethicone (INCI name), such as the product sold by the company Dow Corning under the trade name Silicone DC5225 C or KF-6040 from the company Shin-Etsu.

According to a particularly preferred form, use will be made of a mixture of at least one emulsifier of formula (I) and of at least one emulsifier of formula (II).

Use will be made more particularly of a mixture of PEG-18/PPG-18 Dimethicone and Cetyl PEG/PPG-10/1 Dimethicone and even more particularly of a mixture of (Cyclopentasiloxane (and) PEG-18/PPG-18 Dimethicone) and of Cetyl PEG/PPG-10/1 Dimethicone and Dimethicone or of (Polyglyceryl-4 Stearate and Cetyl PEG/PPG-10 (and) Dimethicone (and) Hexyl Laurate).

The total amount of emulsifiers of formula (I) and/or of emulsifiers of formula (II) in the composition preferably varies at active material contents ranging from 0.3% to 8% by weight and more particularly from 0.5% to 4% by weight relative to the total weight of the composition.

### Non-silicone W/O emulsifiers with an HLB of 3 to 7

According to one particular form of the invention, the compositions may contain, as a replacement for the silicone emulsifiers or in addition, at least one non-silicone, non-ionic water-in-oil emulsifier.

Said emulsifier may be chosen, for example, from non-ionic emulsifiers derived from fatty acids and polyols, alkyl polyglycosides (APGs), sugar esters and mixtures thereof.

Use may in particular be made, as non-ionic emulsifiers derived from fatty acids and polyols, of fatty acid esters of polyols, the fatty acid having in particular a C₈-C₂₄ alkyl chain and the polyols being, for example, glycerol and sorbitan.

Mention may in particular be made, as fatty acid esters of polyols, of isostearic acid esters of polyols, stearic acid esters of polyols, and mixtures thereof, in particular isostearic acid esters of glycerol and/or sorbitan.

Mention may in particular be made, as stearic acid esters of polyols, of the polyethylene glycol esters, such as PEG-30 Dipolyhydroxystearate, for example the product sold under the name Arlacel P135 by the company ICI.

Mention may be made, as glycerol and/or sorbitan esters, for example, of polyglyceryl isostearate, such as the product sold under the name Isolan GI 34 by the company Goldschmidt; sorbitan isostearate, such as the product sold under the name Arlacel 987 by the company ICI; sorbitan glyceryl isostearate, such as the product sold under the name Arlacel 986 by the company ICI, the mixture of sorbitan isostearate and polyglyceryl isostearate (3 mol) sold under the name Arlacel 1690 by the company Uniqema, and mixtures thereof.

The emulsifier can also be chosen from alkylpolyglycosides having an HLB of less than 7, for example those represented by the following general formula (1):

R-O-(G)ₓ (1)

in which R represents a branched and/or unsaturated alkyl radical comprising from 14 to 24 carbon atoms, G represents a reduced sugar comprising 5 or 6 carbon atoms and x denotes a value ranging from 1 to 10 and preferably from 1 to 4, and G in particular denotes glucose, fructose or galactose.

The unsaturated alkyl radical can comprise one or more ethylenic unsaturations and in particular one or two ethylenic unsaturations.

As alkyl polyglycosides of this type, mention may be made of alkyl polyglucosides (G = glucose in formula (I)), and in particular the compounds of formula (I) in which R more particularly represents an oleyl radical (unsaturated C₁₈ radical) or isostearyl radical (saturated C₁₈ radical), G denotes glucose, x is a value ranging from 1 to 2, in particular isostearyl glucoside or oleyl glucoside, and mixtures thereof. This alkyl polyglucoside can be used as a mixture with a coemulsifier, more in particular with a fatty alcohol and in particular a fatty alcohol having the same fatty chain as that of the alkyl polyglucoside, that is to say comprising from 14 to 24 carbon atoms and having a branched and/or unsaturated chain, for example isostearyl alcohol when the alkyl polyglucoside is isostearyl glucoside and oleyl alcohol when the alkyl polyglucoside is oleyl glucoside, optionally in the form of a self-emulsifying composition, as described, for example, in the document WO-A-92/06778. Use may, for example, be made of the mixture of isostearyl glucoside and isostearyl alcohol, sold under the name Montanov WO 18 by the company SEPPIC.

Mention may also be made of succinic-terminated polyolefins, such as esterified succinic-terminated polyisobutylenes and their salts, in particular the diethanolamine salts, such as the products sold under the names Lubrizol 2724, Lubrizol 2722 and Lubrizol 5603 by the company Lubrizol or the commercial product Chemcinnate 2000.

The preferred emulsifier is Polyglyceryl-3 Diisostearate (INCI name) sold under the name Lameform TGI by Cognis.

The non-silicone W/O emulsifier is preferably used:
1) when it is added to the silicone emulsifiers of formula (I) and/or of formula (II) in an amount of active material ranging from 0.1% to 5% and more preferentially from 0.2% to 3% by weight relative to the total weight of the composition;
2) when it is used alone, in an amount of active material ranging from 1% to 8% by weight and more particularly from 2% to 6% relative to the total weight of the composition.

The non-silicone W/O emulsifiers with an HLB ranging from 3 to 7 may be present in the cosmetic composition according to the invention in a content ranging from 0.5% to 6% by weight and preferably in a content ranging from 2% to 5% by weight relative to the total weight of the composition.

A combination of at least one silicone emulsifier of formula (I) and/or at least one silicone emulsifier of formula (II) with at least one non-silicone W/O emulsifier with an HLB ranging from 3 to 7 will preferentially be used.

### Oily phase

The compositions according to the invention may contain at least one water-immiscible organic liquid phase, known as oily phase. This phase generally comprises one or more hydrophobic compounds that make said phase water-immiscible. Said phase is liquid (in the absence of structuring agent) at ambient temperature (20-25°C). Preferentially, the water-immiscible organic liquid phase in accordance with the invention generally comprises at least one volatile oil and/or one non-volatile oil and optionally at least one structuring agent.

The term "oil" is intended to mean a fatty substance that is liquid at ambient temperature (25°C) and atmospheric pressure (760 mmHg, i.e. 10⁵ Pa). The oil can be volatile or non-volatile.

For the purpose of the invention, the term "volatile oil" is intended to mean an oil which is capable of evaporating on contact with the skin or with the keratin fibre in less than one hour, at ambient temperature and atmospheric pressure. The volatile oils of the invention are volatile cosmetic oils which are liquid at ambient temperature and which have a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is intended to mean an oil which remains on the skin or the keratin fibre at ambient temperature and atmospheric pressure for at least several hours and which has in particular a vapour pressure of less than 10⁻³ mmHg (0.13 Pa).

The oil can be chosen from any physiologically acceptable oil and in particular cosmetically acceptable oils, in particular mineral, animal, vegetable or synthetic oils; in particular volatile or non-volatile hydrocarbon-based oils and/or silicone oils and/or fluoro oils, and mixtures thereof.

More specifically, the term "hydrocarbon-based oil" is intended to mean an oil mainly comprising carbon and hydrogen atoms and optionally one or more functions chosen from hydroxyl, ester, ether and carboxylic functions. Generally, the oil has a viscosity of from 0.5 to 100 000 mPa.s, preferably from 50 to 50 000 mPa.s and more preferably from 100 to 300 000 mPa.s.

Mention may be made, as examples of volatile oil which can be used in the invention, of:
- volatile hydrocarbon-based oils chosen from hydrocarbon-based oils containing from 8 to 16 carbon atoms, and in particular C8-C16 isoalkanes of petroleum origin (also known as isoparaffins), for instance isododecane (also known as 2,2,4,4,6-pentamethylheptane), isodecane and isohexadecane, for example the oils sold under the trade names Isopar or Permethyl, branched C8-C16 esters and isohexyl neopentanoate, and mixtures thereof. Use may also be made of other volatile hydrocarbon-based oils, such as petroleum distillates, in particular those sold under the name Shell Solt by the company Shell; and volatile linear alkanes, such as those described in patent application DE10 2008 012 457 from Cognis;
- volatile silicones, for instance volatile linear or cyclic silicone oils, in particular those with a viscosity ≤ 8 centistokes (8×10⁻⁶ m²/s) and in particular containing from 2 to 7 silicon atoms, these silicones optionally comprising alkyl or alkoxy groups containing from 1 to 10 carbon atoms. Mention may in particular be made, as volatile silicone oils which can be used in the invention, of cyclohexasiloxane, octamethylcyclotetrasiloxane, decamethylcclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane or dodecamethylpentasiloxane;
- and mixtures thereof.

Mention may also be made of the volatile linear alkyltrisiloxane oils of general formula (I): 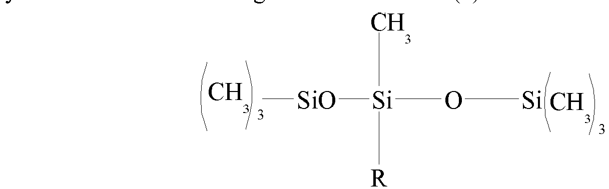 where R represents an alkyl group comprising from 2 to 4 carbon atoms, one or more hydrogen atoms of which can be replaced by a fluorine or chlorine atom.

Mention may be made, among the oils of general formula (I), of:
3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Mention may be made, as examples of non-volatile oil which can be used in the invention, of:
- hydrocarbon-based oils of animal origin, such as perhydrosqualene;
- vegetable hydrocarbon-based oils, such as liquid triglycerides of fatty acids having 4 to 24 carbon atoms, such as heptanoic or octanoic acid triglycerides, or else wheat germ oil, olive oil, sweet almond oil, palm oil, rapeseed oil, cottonseed oil, alfalfa oil, poppy seed oil, pumpkin seed oil, cucumber oil, blackcurrant oil, evening primrose oil, millet oil, barley oil, quinoa oil, rye oil, safflower oil, candlenut oil, passionflower oil, musk rose oil, sunflower oil, maize oil, soybean oil, marrow oil, grape seed oil, sesame oil, hazelnut oil, apricot oil, macadamia oil, castor oil, avocado oil, caprylic/capric acid triglycerides, such as those sold by Stearineries Dubois or those sold under the names Miglyol 810, 812 and 818 by Dynamit Nobel, jojoba oil or shea butter oil;
- linear or branched hydrocarbons of mineral or synthetic origin, such as liquid paraffins and their derivatives, petroleum jelly, polydecenes, polybutenes, hydrogenated polyisobutene, such as Parleam, or squalane;
- synthetic ethers containing from 10 to 40 carbon atoms;
- synthetic esters, in particular of fatty acids, for instance the oils of formula R₁COOR₂ in which R₁ represents a linear or branched higher fatty acid residue containing from 1 to 40 carbon atoms and R₂ represents a hydrocarbon-based chain, which is in particular branched, containing from 1 to 40 carbon atoms, with R₁ + R₂ ≥ 10, for instance purcellin oil (cetostearyl octanoate), isononyl isononanoate, isopropyl myristate, isopropyl palmitate, C₁₂-C₁₅ alkyl benzoates, hexyl laurate, diisopropyl adipate, isononyl isononanoate, 2-ethylhexyl palmitate, 2-octyldodecyl stearate, 2-octyldodecyl erucate, isostearyl isostearate or tridecyl trimellitate; alcohol or polyalcohol octanoates, decanoates or ricinoleates, for instance propylene glycol dioctanoate; hydroxylated esters, for instance isostearyl lactate, octyl hydroxystearate, octyldodecyl hydroxystearate, diisostearyl malate, triisocetyl citrate, and fatty alcohol heptanoates, octanoates or decanoates; polyol esters, for instance propylene glycol dioctanoate, neopentyl glycol diheptanoate or diethylene glycol diisononanoate; and pentaerythritol esters, for instance pentaerythrityl tetraisostearate;
- fatty alcohols which are liquid at ambient temperature and which comprise a branched and/or unsaturated carbon-based chain having from 12 to 26 carbon atoms, such as octyldodecanol, isostearyl alcohol, 2-butyloctanol, 2-hexyldecanol, 2-undecylpentadecanol or oleyl alcohol;
- higher fatty acids, such as oleic acid, linoleic acid or linolenic acid;
- carbonates;
- acetates;
- citrates;
- fluoro oils which are optionally partially hydrocarbon-based and/or silicone-based, for example fluorosilicone oils, fluoropolyethers or fluorinated silicones, such as described in the document EP-A-847 752;
- silicone oils, such as non-volatile linear or cyclic polydimethylsiloxanes (PDMSs); polydimethylsiloxanes comprising alkyl, alkoxy or phenyl groups which are pendent or at the end of the silicone chain, which groups have from 2 to 24 carbon atoms; or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates, and
- mixtures thereof.

According to one particular preferred form of the invention, the compositions contain at least one volatile hydrocarbon-based oil and/or at least one volatile silicone oil.

The total amount of volatile oil will preferably be from 10% to 40% by weight relative to the total composition.

The preferential volatile oils are chosen from isododecane, isodecane, isohexadecane, undecane/tridecane mixtures such as those of document DE 10 2008 012 457, cyclohexasiloxane, volatile dimethicones, and mixtures thereof.

### Structuring agent

The compositions according to the invention comprising an oily phase can additionally contain at least one or more structuring agents for said oily phase other than the ethylenic polymer containing at least one carbosiloxane dendrimer-based unit, which can preferably be chosen from waxes, pasty compounds, inorganic or organic lipophilic gelling agents, and mixtures thereof.

It is understood that the amount of these compounds can be adjusted by those skilled in the art so as not to harm the effect desired in the context of the present invention.

### Wax(es)

The wax is generally a lipophilic compound which is solid at ambient temperature (25°C), which exhibits a reversible solid/liquid change in state and which has a melting point of greater than or equal to 30°C which can range up to 200°C and in particular up to 120°C.

In particular, the waxes suitable for the invention can exhibit a melting point of greater than or equal to 45°C and in particular of greater than or equal to 55°C.

For the purpose of the invention, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold under the name MDSC 2920® by the company TA Instruments.

The measurement protocol is as follows:
A sample of 5 mg of wax placed in a crucible is subjected to a first temperature rise ranging from -20°C to 100°C, at a heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation in the difference in power absorbed by the empty crucible and by the crucible containing the sample of wax is measured as a function of the temperature. The melting point of the compound is the temperature value corresponding to the top of the peak of the curve representing the variation in the difference in power absorbed as a function of the temperature.

The waxes capable of being used in the compositions according to the invention are chosen from waxes which are solid at ambient temperature and which are of animal, vegetable, mineral or synthetic origin, and mixtures thereof.

As illustrations of waxes that are suitable for the invention, mention may be made in particular of hydrocarbon-based waxes, for instance beeswax, lanolin wax, Chinese insect waxes, rice bran wax, carnauba wax, candelilla wax, ouricury wax, esparto grass wax, berry wax, shellac wax, Japan wax and sumach wax; montan wax, orange wax and lemon wax, refined sunflower wax sold under the name Sunflower Wax® by Koster Keunen, microcrystalline waxes, paraffins and ozokerite; polyethylene waxes, the waxes obtained by Fischer-Tropsch synthesis and waxy copolymers, and also esters thereof.

Mention may also be made of waxes obtained by catalytic hydrogenation of animal or vegetable oils containing linear or branched C₈-C₃₂ fatty chains. Mention may in particular be made, among these waxes, of isomerized jojoba oil, such as the trans-isomerized partially hydrogenated jojoba oil manufactured or sold by the company Desert Whale under the commercial reference Iso-Jojoba-50®, hydrogenated sunflower oil, hydrogenated castor oil, hydrogenated coconut oil, hydrogenated lanolin oil and bis(1,1,1-trimethylolpropane) tetrastearate, sold under the name Hest 2T-4S® by the company Heterene.

Mention may also be made of silicone waxes (C₃₀₋₄₅ alkyl dimethicone) or fluorinated waxes.

Use may also be made of the waxes obtained by hydrogenation of castor oil esterified with cetyl alcohol, sold under the names Phytowax Castor 16L64® and 22L73® by the company Sophim. Such waxes are described in patent application FR-A-2 792 190.

A wax that may be used is a C₂₀-C₄₀ alkyl (hydroxystearyloxy)stearate (the alkyl group containing from 20 to 40 carbon atoms), alone or as a mixture.

Such a wax is sold in particular under the names Kester Wax K 82 P®, Hydroxypolyester K 82 P® and Kester Wax K 80 P® by the company Koster Keunen.

As microwaxes that may be used in the compositions according to the invention, mention may be made in particular of carnauba microwaxes, such as the product sold under the name MicroCare 350® by the company Micro Powders, synthetic microwaxes, such as the product sold under the name MicroEase 114S® by the company Micro Powders, microwaxes consisting of a mixture of carnauba wax and polyethylene wax, such as the products sold under the names Micro Care 300® and 310® by the company Micro Powders, microwaxes consisting of a mixture of carnauba wax and of synthetic wax, such as the product sold under the name Micro Care 325® by the company Micro Powders, polyethylene microwaxes, such as the products sold under the names Micropoly 200®, 220®, 220L® and 250S® by the company Micro Powders, the commercial products Performalene 400 Polyethylene® and Performalene 500-L Polyethylene® from New Phase Technologies, Performalene 655 Polyethylene or paraffin waxes, for instance the wax having the INCI name Microcrystalline Wax® and Synthetic Wax and sold under the trade name Microlease® by the company Sochibo; polytetrafluoroethylene microwaxes, such as those sold under the names Microslip 519® and 519 L® by the company Micro Powders.

The composition according to the invention may preferably comprise a content of wax(es) ranging from 3% to 20% by weight relative to the total weight of the composition, in particular from 5% to 15% by weight and more particularly from 6% to 15%.

According to one specific form of the invention, in the context of anhydrous solid compositions in the stick form, use may be made of polyethylene microwaxes in the form of crystallites with an aspect ratio at least equal to 2 and with a melting point ranging from 70°C to 110°C and preferably from 70°C to 100°C, in order to reduce or even eliminate the presence of strata in the solid composition.

These crystallites in needle form and in particular their dimensions can be characterized visually according to the following method.

The wax is deposited on a microscope slide, which is placed on a hotplate. The slide and the wax are heated to a temperature generally at least 5°C higher than the melting point of the wax or of the mixture of waxes under consideration. At the end of melting, the liquid thus obtained and the microscope slide are allowed to cool in order to solidify. Observation of the crystallites is performed using a Leica DMLB100® optical microscope, with an objective lens selected as a function of the size of the objects to be viewed, and under polarized light. The dimensions of the crystallites are measured using image analysis software, such as those sold by the company Microvision.

The crystallite polyethylene waxes in accordance with the invention preferably have an average length ranging from 5 to 10 µm. The term "average length" denotes the dimension given by the statistical particle size distribution to half the population, referred to as D50.

Use will be made more particularly of a mixture of Performalene 400 Polyethylene® and Performalene 500-L Polyethylene® waxes from New Phase Technologies.

### Pasty compounds

For the purpose of the present invention, the term "pasty compound" is intended to mean a lipophilic fatty compound which exhibits a reversible solid/liquid change of state, which exhibits, in the solid state, an anisotropic crystal arrangement and which comprises, at the temperature of 23°C, a liquid fraction and a solid fraction.

The pasty compound is preferably chosen from synthetic compounds and compounds of vegetable origin. A pasty compound can be obtained by synthesis from starting materials of vegetable origin.

The pasty compound can advantageously be chosen from:
- lanolin and its derivatives,
- polymeric or non-polymeric silicone compounds,
- polymeric or non-polymeric fluorinated compounds,
- vinyl polymers, in particular:
- olefin homopolymers,
- olefin copolymers,
- hydrogenated diene homopolymers and copolymers,
- linear or branched oligomers, homopolymers or copolymers of alkyl (meth)acrylates preferably containing a C₈-C₃₀ alkyl group,
- homo- and copolymeric oligomers of vinyl esters having C₈-C₃₀ alkyl groups, and
- homo- and copolymeric oligomers of vinyl ethers having C₈-C₃₀ alkyl groups,
- liposoluble polyethers resulting from polyetherification between one or more C₂-C₁₀₀, preferably C₂-C₅₀, diols,
- esters,
- mixtures thereof.

Preference is given in particular, among the esters, to:
- esters of a glycerol oligomer, in particular diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, in particular such as those sold under the brand name Softisan 649® by the company Sasol,
- the arachidyl propionate sold under the brand name Waxenol 801 by Alzo,
- phytosterol esters,
- fatty acid triglycerides and derivatives thereof,
- pentaerythritol esters,
- non-crosslinked polyesters resulting from polycondensation between a linear or branched C₄-C₅₀ dicarboxylic acid or polycarboxylic acid and a C₂-C₅₀ diol or polyol,
- aliphatic esters of an ester resulting from the esterification of an aliphatic hydroxycarboxylic acid ester by an aliphatic carboxylic acid,
- polyesters resulting from the esterification, by a polycarboxylic acid, of an aliphatic hydroxycarboxylic acid ester, said ester comprising at least two hydroxyl groups, such as the products Risocast DA-H® and Risocast DA-L®,
- esters of a diol dimer and of a diacid dimer, where appropriate, esterified on their free alcohol or acid function(s) with acid or alcohol radicals, such as Plandool-G®,
- mixtures thereof.

The choice will preferably be made, among pasty compounds of vegetable origin, of a mixture of soya sterols and of oxyethylenated (5 EO)/oxypropylenated (5 PO) pentaerythritol sold under the reference Lanolide by the company Vevy.

### Lipophilic gelling agents

### Inorganic gelling agents

Inorganic lipophilic gelling agents that may be mentioned include optionally modified clays, for instance hectorites modified with a C₁₀ to C₂₂ ammonium chloride, for instance hectorite modified with distearyldimethylammonium chloride, for instance the product sold under the name Bentone 38V® by the company Elementis.

### Organic gelling agents

The polymeric organic lipophilic gelling agents are, for example, partially or totally crosslinked elastomeric organopolysiloxanes of three-dimensional structure, for instance those sold under the names KSG6®, KSG16® and KSG18® by the company Shin-Etsu, Trefil E-505C® or Trefil E-506C® by the company Dow Corning, Gransil SR-CYC®, SR DMF10®, SR-DC556®, SR 5CYC gel®, SR DMF 10 gel® and SR DC 556 gel® by the company Grant Industries and SF 1204® and JK 113® by the company General Electric; ethylcellulose, for instance the product sold under the name Ethocel® by the company Dow Chemical; galactomannans comprising from one to six and in particular from two to four hydroxyl groups per saccharide, substituted with a saturated or unsaturated alkyl chain, for instance guar gum alkylated with C₁ to C₆, and in particular C₁ to C₃, alkyl chains, and mixtures thereof.

Mention may also be made, as lipophilic gelling agent, of polymers with a weight-average molecular weight of less than 100 000 comprising a) a polymer backbone having hydrocarbon-based repeat units provided with at least one heteroatom and optionally b) at least one optionally functionalized pendent fatty chain and/or at least one optionally functionalized terminal fatty chain having from 6 to 120 carbon atoms and being bonded to these hydrocarbon-based units, such as described in applications WO-A-02/056847 and WO-A-02/47619, in particular polyamide resins (in particular comprising alkyl groups having from 12 to 22 carbon atoms), such as those described in US-A-5 783 657.

Mention may also be made, among the lipophilic gelling agents which can be used in the compositions according to the invention, of esters of dextrin and of fatty acid, such as dextrin palmitates, in particular such as those sold under the names Rheopearl TL® and Rheopearl KL® by the company Chiba Flour.

Use may also be made of silicone polyamides of the polyorganosiloxane type, such as those described in the documents US-A-5 874 069, US-A-5 919 441, US-A-6 051 216 and US-A-5 981 680.

These silicone polymers can belong to the following two families:
- polyorganosiloxanes comprising at least two groups capable of establishing hydrogen bond interactions, these two groups being situated in the chain of the polymer, and/or
- polyorganosiloxanes comprising at least two groups capable of establishing hydrogen bond interactions, these two groups being situated on grafts or branchings.

### Additives

The cosmetic compositions according to the invention may also comprise one or more cosmetic adjuvants chosen from organic powders, softeners, antioxidants, opacifiers, stabilizers, moisturizers, vitamins, bactericides, preserving agents, polymers, fragrances, thickeners or suspending agents, propellants or any other ingredient usually used in cosmetics for this type of application.

Needless to say, those skilled in the art will take care to select this or these optional additional compound(s) such that the advantageous properties intrinsically associated with the cosmetic composition in accordance with the invention are not, or are not substantially, adversely affected by the envisaged addition(s).

### Organic powder

According to one particular form of the invention, the compositions of the invention may also contain one or more organic powders.

In the present patent application, the term "organic powder" is intended to mean any solid which is insoluble in the medium at ambient temperature (25°C).

As organic powders that may be used in the composition of the invention, examples that may be mentioned include polyamide particles and in particular those sold under the Orgasol® names by the company Atochem; nylon-6,6 fibres, in particular the polyamide fibres sold by Etablissements P Bonte under the name Polyamide 0.9 Dtex 0.3 mm® (INCI name: Nylon-6,6® or Polyamide-6,6) with a mean diameter of 6 µm, a weight of about 0.9 dtex and a length ranging from 0.3 mm to 1.5 mm; polyethylene powders; microspheres based on acrylic copolymers, such as those made of ethylene glycol dimethacrylate/lauryl methacrylate copolymer, sold by the company Dow Corning under the name Polytrap®; polymethyl methacrylate microspheres, sold under the name Microsphere M-100® by the company Matsumoto or under the name Covabead LH85® by the company Wackherr; hollow polymethyl methacrylate microspheres (particle size: 6.5-10.5 µm) sold under the name Ganzpearl GMP 0800 by Ganz Chemical; methyl methacrylate/ethylene glycol dimethacrylate copolymer microbeads (size: 6.5-10.5 µm) sold under the name Ganzpearl GMP 0820® by Ganz Chemical or Microsponge 5640® by the company Amcol Health & Beauty Solutions; ethylene-acrylate copolymer powders, such as those sold under the name Flobeads® by the company Sumitomo Seika Chemicals; expanded powders such as hollow microspheres and in particular microspheres formed from a terpolymer of vinylidene chloride, acrylonitrile and methacrylate and sold under the name Expancel® by the company Kemanord Plast under the references 551 DE 12® (particle size of about 12 µm and mass per unit volume of 40 kg/m³), 551 DE 20® (particle size of about 30 µm and mass per unit volume of 65 kg/m³), 551 DE 50® (particle size of about 40 µm), or the microspheres sold under the name Micropearl F 80 ED® by the company Matsumoto; powders of natural organic materials such as powders of starch, in particular of crosslinked or non-crosslinked corn, wheat or rice starch, such as the powders of starch crosslinked with octenylsuccinic anhydride, sold under the name Dry-Flo® by the company National Starch; silicone resin microbeads such as those sold under the name Tospearl® by the company Toshiba Silicone, in particular Tospearl 240®; amino acid powders such as the lauroyllysine powder sold under the name Amihope LL-11® by the company Ajinomoto; particles of wax microdispersion, which preferably have mean sizes of less than 1 µm and in particular ranging from 0.02 µm to 1 µm, and which are formed essentially from a wax or a mixture of waxes, such as the products sold under the name Aquacer® by the company Byk Cera, and in particular: Aquacer 520 (mixture of synthetic and natural waxes), Aquacer 514 or 513® (polyethylene wax), Aquacer 511 (polymeric wax), or such as the products sold under the name Jonwax 120 by the company Johnson Polymer (mixture of polyethylene wax and paraffin wax) and under the name Ceraflour 961® by the company Byk Cera (micronized modified polyethylene wax); and mixtures thereof.

According to one particularly preferred form of the invention, the composition according to the invention also contains polyamide particles and in particular polyamide-12 (nylon-12) particles or polyamide-6 (nylon-6) particles.

More preferentially, the composition according to the invention also comprises one or more polyamide-12 (nylon-12) particles, such as those sold under the trade name Orgasol 2002-EXD NAT COS by the company Arkema.

The polyamide particles used in the invention may be those sold under the name Orgasol by the company Atochem. The process for obtaining these particles is, for example, the one described in document FR 2 619 385 or in document EP 303530. These polyamide particles are moreover known, according to their various physicochemical properties, as "polyamide 12" or "polyamide 6".

### Inorganic powders

According to one particular form of the invention, the compositions according to the invention may also contain one or more inorganic powders chosen from silica particles, perlite particles, talc and kaolin.

### Thickeners

The thickeners can be chosen from carboxyvinyl polymers, such as the Carbopols (Carbomers) and the Pemulens (acrylate/C₁₀-C₃₀ alkyl acrylate copolymer); polyacrylamides, such as, for example, the crosslinked copolymers sold under the names Sepigel 305 (CTFA name: polyacrylamide/C₁₃₋₁₄ isoparaffin/Laureth 7) or Simulgel 600 (CTFA name: acrylamide/sodium acryloyldimethyltaurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Hoechst under the trade name Hostacerin AMPS® (CTFA name: ammonium polyacryloyldimethyltaurate) or Simulgel 800® sold by the company SEPPIC (CTFA name: sodium polyacryloyldimethyltaurate/polysorbate 80/sorbitan oleate); copolymers of 2-acrylamido-2-methylpropanesulfonic acid and of hydroxyethyl acrylate, for instance Simulgel NS and Sepinov EMT 10® sold by the company SEPPIC; cellulose derivatives such as hydroxyethyl cellulose or cetyl hydroxyethyl cellulose; polysaccharides and in particular gums such as xanthan gum and hydroxypropyl guar gums; silicas, for instance Bentone Gel MIO® sold by the company NL Industries or Veegum Ultra® sold by the company Polyplastic.

The thickeners may also be cationic, for instance Polyquaternium-37 sold under the name Salcare SC95® (Polyquaternium-37 (and) Mineral Oil (and) PPG-1 Trideceth-6) or Salcare SC96® (Polyquaternium-37 (and) Propylene Glycol Dicaprylate/Dicaprate (and) PPG-1 Trideceth-6) or other crosslinked cationic polymers, for instance those of the CTFA name Ethyl Acrylate/Dimethylaminoethyl Methacrylate Cationic Copolymer In Emulsion.

### Suspending agents

In order to improve the homogeneity of the product, use may additionally be made of one or more suspending agents which are preferably chosen from hydrophobic modified montmorillonite clays, such as hydrophobic modified bentonites or hectorites. Examples that may be mentioned include the product Stearalkonium Bentonite (CTFA name) (product of reaction of bentonite and the quaternary ammonium stearalkonium chloride) such as the commercial product sold under the name Tixogel MP 250 by the company Sud Chemie Rheologicals, United Catalysts Inc. or the product Disteardimonium Hectorite (CTFA name) (product of reaction of hectorite and distearyldimonium chloride) sold under the name Bentone 38 or Bentone Gel by the company Elementis Specialities.

Other suspending agents can be used, in this case in hydrophilic (aqueous and/or ethanolic) media. They can be derivatives of cellulose, xanthan, guar, starch, locust bean or agar agar.

The suspending agents are preferably present in amounts ranging from 0.1% to 5% by weight and more preferentially from 0.2% to 2% by weight, relative to the total weight of the composition.

The amounts of these various constituents which can be present in the cosmetic composition according to the invention are those conventionally used in compositions for the treatment of perspiration.

The antiperspirant cosmetic composition according to the invention may also contain one or more deodorants.

The term "deodorant active agent" is intended to mean any substance that is capable of reducing, masking or absorbing human body odour and in particular underarm odour.

The deodorant active agents may be bacteriostatic agents or bactericides that act on underarm odour microorganisms, such as 2,4,4'-trichloro-2'-hydroxydiphenyl ether (®Triclosan), 2,4-dichloro-2'-hydroxydiphenyl ether, 3',4',5'-trichlorosalicylanilide, 1-(3',4'-dichlorophenyl)-3-(4'-chlorophenyl)urea (®Triclocarban) or 3,7,11-trimethyldodeca-2,5,10-trienol (®Farnesol); quaternary ammonium salts such as cetyltrimethylammonium salts, cetylpyridinium salts, DPTA (1,3-diaminopropanetetraacetic acid), 1,2-decanediol (Symclariol from the company Symrise), glycerol derivatives, for instance caprylic/capric glycerides (Capmul MCM® from Abitec), glyceryl caprylate or caprate (Dermosoft GMCY® and Dermosoft GMC®, respectively from Straetmans), polyglyceryl-2 caprate (Dermosoft DGMC® from Straetmans), and biguanide derivatives, for instance polyhexamethylene biguanide salts; chlorhexidine and salts thereof; 4-phenyl-4,4-dimethyl-2-butanol (Symdeo MPP® from Symrise); zinc salts such as zinc salicylate, zinc gluconate, zinc pidolate, zinc sulfate, zinc chloride, zinc lactate or zinc phenolsulfonate; salicylic acid and derivatives thereof such as 5-n-octanoylsalicylic acid.

The deodorant active agents may be odour absorbers such as zinc ricinoleates or sodium bicarbonate; metallic or silver or silver-free zeolites, or cyclodextrins and derivatives thereof. They may also be chelating agents such as Dissolvine GL-47-S® from Akzo Nobel, EDTA and DPTA. It may also be a polyol such as glycerol or 1,3-propanediol (Zemea Propanediol sold by Dupont Tate and Lyle BioProducts), or else an enzyme inhibitor such as triethyl citrate; or alum.

In the event of incompatibility or to stabilize them, for example, some of the active agents mentioned above may be incorporated into spherules, in particular ionic or non-ionic vesicles and/or nanoparticles (nanocapsules and/or nanospheres).

The deodorant active agents may be present in the cosmetic composition according to the invention in a concentration of from 0.01% to 15% by weight relative to the total weight of the composition.

The composition according to the invention may be in pressurized form such as a spray or an aerosol device and may in this regard contain the ingredients generally used in products of this type which are well known to those skilled in the art.

The compositions according to the invention can also be pressurized and be packaged in an aerosol device made up of:
(A) a container comprising an antiperspirant composition as defined previously,
(B) at least one propellant and one means for dispensing said aerosol composition.

The propellants generally used in products of this type and that are well known to those skilled in the art are, for instance, dimethyl ether (DME); volatile hydrocarbons such as n-butane, propane, isobutane and mixtures thereof, optionally with at least one chlorohydrocarbon and/or fluorohydrocarbon; among these, mention may be made of the compounds sold by the company DuPont de Nemours under the names Freon® and Dymel®, and in particular mono fluorotrichloromethane, difluorodichloromethane, tetrafluorodichloroethane and 1,1-difluoroethane sold in particular under the trade name Dymel 152 A® by the company DuPont. Use may also be made, as propellant, of carbon dioxide gas, nitrous oxide, nitrogen or compressed air.

The compositions as defined previously and the propellant(s) may be in the same compartment or in different compartments in the aerosol container. According to the invention, the concentration of propellant generally varies from 5% to 95% by weight pressurized and more preferentially from 50% to 85% by weight, relative to the total weight of the pressurized composition.

The invention also relates to a process for cosmetic treatment of human perspiration, and optionally of underarm odour, consisting in applying an effective amount of the cosmetic composition as described previously to the surface of the skin.

The cosmetic composition in accordance with the invention may be applied several times to the surface of the skin.

In particular, the process for cosmetic treatment according to the invention consists in applying an effective amount of the cosmetic composition as described above to the surface of the armpits.

The examples which follow illustrate the present invention.

### Examples:

### 1. Compositions tested

The following compositions are prepared from the ingredients indicated in the following table. The percentages are expressed as starting material percentages.

| Phase | Ingredients | T | A Inv | B Comp | C Comp | D Inv |
|---|---|---|---|---|---|---|
| A | Dimethicone ⁽¹⁾ | 20 | - | - | - | - |
| A | Dimethicone and dimethiconol ⁽²⁾ | 2 | - | - | - | - |
| A | Lauryl PEG-9 Polydimethylsiloxye thyl dimethicone ⁽³⁾ | 1 | - | - | - | - |
| A1 | Isohexadecane ⁽⁴⁾ | - | 5 | 8.6 | 5 | 4.1 |
| A1 | Hydrogenated styrene/ isoprene copolymer ⁽⁵⁾ | - | 0.9 | - | - | 0.45 |
| A2 | Disteardimethylam monium hectorite and propylene carbonate ⁽⁶⁾ | - | 8 | 8 | 8 | 4 |
| A2 | Acrylate/polytrimet hylsiloxy methacrylate copolymer ⁽⁷⁾ | - | 13 | - | - | 6.5 |
| A2 | Dimethicone PEG/ PPG-18/ 18 Dimethicone ⁽⁸⁾ | - | 2 | 2 | 2 | 2 |
| A2 | Isododecane ⁽⁹⁾ | - | 9.45 | 16.15 | 9.45 | 5.625 |
| A2 | Undecane and tridecane ⁽¹⁰⁾ | - | 5 | 8.6 | 5 | - |
| A2 | Polyglyceryl-3-diisostearate ⁽¹¹⁾ | - | 1 | 1 | 1 | 1 |
| A2 | PEG-10 dimethicone ⁽¹²⁾ | - | 1 | 1 | 1 | 1 |
| A3 | Nylon-12 ⁽¹³⁾ | - | 7 | 7 | 7 | 3.5 |
| B | Aluminium hydrochloride ⁽¹⁴⁾ | 20 (10%) AM) | 20 (10% ) AM) | 20 (10%) AM) | 20 (10%) AM) | 20 (10%) AM) |
| B | Methylparaben ⁽¹⁵⁾ | - | 0.25 | 0.25 | 0.25 | 0.25 |
| B | Caprylyl glycol ⁽¹⁶⁾ | - | 0.3 | 0.3 | 0.3 | 0.3 |
| B | Water | 54.33 | Qs 100 | 23.6 | 37.5 | Qs 100 |
| c | Tetrasodium glutamate diacetate ⁽¹⁷⁾ | 1.667 | - | - | - | - |
| D | Phenoxyethanol | 1 | 0.5 | 0.5 | 0.5 | 0.5 |
| E | Alcohol | | 3 | 3 | 3 | 3 |

| | | | | | | |
|---|---|---|---|---|---|---|
| (1) sold under the name Belsil DM 10 by the company Wacker (2) sold under the name Mirasil D-DML LV by the company Bluestar (3) sold under the name KF-6038 by the company Shin Etsu (4) sold under the name Isohexadecane by the company Ineos (5) sold under the name Kraton G1701 EU by the company Kraton Polymers (6) sold under the name Bentone GEL ISD V by the company Elementis (7) sold under the name Dow Corning FA 4002 ID Silicone Acrylate by the company Dow Corning (8) sold under the name X-22-6711D by the company Shin Etsu (9) sold under the name Isododecane by the company Ineos (10) Undecane and tridecane mixture according to Example 1 or 2 of document DE10 2008 012 457. (11) sold under the name Lameform TGI by the company BASF (12) sold under the name KF-6017 by the company Shin Etsu (13) sold under the name Orgasol 2002 EXD NAT COS by the company Arkema (14) sold under the name Chlorhydrol 50 by the company Summitreheis (15) sold under the name Methyl Paraben by the company Sharon Laboratories (16) sold under the name Dermosoft octiol by the company Dr Straetmans (17) sold under the name Dissolvine GL-47-S by the company Akzo Nobel | | | | | | |

### 2. Procedure - preparation of the compositions:

### 2.1. Preparation of the control composition (T)

Phase A is mixed with a Rayneri blender. Phase B is then added slowly to the phase and stirring is then carried out for 15 minutes. Phases C and D are then added.

### 2.2. Preparation of composition (A) according to the invention

The aqueous phase is first prepared by mixing together phases (B) and (D). The mixture obtained (B+D) is heated at a temperature of 85°C until solubilization of the two phases. The mixture is then cooled to ambient temperature.

The hydrogenated isoprene/styrene copolymer is swollen in isohexadecane (phase A1) for 1 hour at 85°C with stirring using a Moritz homogenizer at a stirring speed of 4000 rpm. The resulting product is cooled to ambient temperature and phases (A2) and then (A3) are subsequently added to phase (A1). The cooled mixture (B+D) is then slowly added to phase (A1) and stirring is carried out for fifteen minutes.

Denatured alcohol is then added (phase E).

### 2.3. Preparation of comparative compositions (B) and (C)

The aqueous phase is first prepared by mixing together phases (B) and (D). The mixture obtained (B+D) is heated at a temperature of 85°C until solubilization of the two phases. The mixture is then cooled to ambient temperature.

The hydrogenated isoprene/styrene copolymer is swollen in isohexadecane (phase A1) for 1 hour at 85°C with stirring using a Moritz homogenizer. The resulting product is cooled to ambient temperature and phases (A2) and then (A3) are subsequently added to phase (A1). The cooled mixture (B+D) is then slowly added to phase (A1) and stirring is carried out for fifteen minutes.

Compositions (T), (A), (B) and (C) having an aluminium hydrochloride content of approximately 10% by weight are obtained.

### 3. Preparation of an aerosol composition containing 3% AM of aluminium hydrochloride

| | T + Propellant | A + Propellant | B + Propellant | C + Propellant | D + Propellant |
|---|---|---|---|---|---|
| T | 30 | | | | |
| A | | 30 | | | |
| B | | | 30 | | |
| C | | | | 30 | |
| D | | | | | 30 |
| Isobutane | 70 | 70 | 70 | 70 | 70 |

### 3. Results regarding anti-transfer effectiveness

### 3.1. Protocol for measuring the anti-transfer effectiveness

The composition to be studied is applied to an article made of imitation leather, sold under the name Supplale® by the company Idemitsu Technofine, which is adhesively bonded to a smooth black sheet of 170 grams.

The composition is applied with a 100-micron automatic spreader in order to obtain a homogeneous and reproducible film.

After 24 hours, a black cotton fabric is placed on the article made of imitation leather. A weight of 2 kilograms is then applied to the black fabric so that the fabric becomes impregnated with the composition.

The weight is run up and down the entire length of the film while pulling the fabric taught.

The fabric is scanned with a scanner sold under the name Scanner Epson perfection V500 Photo.

The grey level of the scans is then analysed using an imageJ software which has a grey level ranging from 0 to 255.

### 3.2. Results

| | Fabric without product | T (Control) | A (Inv) | B (Comp) | C (Comp) | D (Inv) |
|---|---|---|---|---|---|---|
| Grey level | 38+/-0 | 77 ± 3 | 42±1 | 79±1 | 71±1 | 41±1 |

It is noted that the grey level is lower for compositions (A) and (D) according to the invention. Furthermore, the values are very close to that of the fabric alone. This means that compositions (A) and (D) according to the invention transfer less than compositions (T), (B) and (C).

## Claims

1. Antiperspirant cosmetic composition in water-in-oil emulsion form, comprising, in a cosmetically acceptable medium, one or more antiperspirant active agents, one or more vinyl polymers containing at least one carbosiloxane dendrimer-based unit, and one or more olefin copolymers, said antiperspirant cosmetic composition being in an aerosol form.

2. Cosmetic composition according to Claim 1, **characterized in that** the antiperspirant active agent is an aluminium salt.

3. Cosmetic composition according to Claim 1 or 2, **characterized in that** the aluminium salt is chosen from aluminium chlorohydrate, aluminium chlorohydrex, aluminium chlorohydrex PEG, aluminium chlorohydrex PG, aluminium dichlorohydrate, aluminium dichlorohydrex PEG, aluminium dichlorohydrex PG, aluminium sesquichlorohydrate, aluminium sesquichlorohydrex PEG, aluminium sesquichlorohydrex PG, alum salts, aluminium sulfate, aluminium zirconium octachlorohydrate, aluminium zirconium pentachlorohydrate, aluminium zirconium tetrachlorohydrate and aluminium zirconium trichlorohydrate.

4. Cosmetic composition according to any one of Claims 1 to 3, **characterized in that** the vinyl polymer containing at least one carbosiloxane dendrimer-based unit has a molecular side chain containing a carbosiloxane dendrimer structure represented by the following general formula (I): 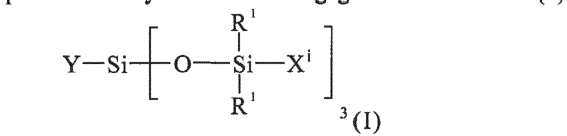 in which R¹ represents an aryl group or an alkyl group containing from 1 to 10 carbon atoms, and X' represents a silylalkyl group which, when i = 1, corresponds to formula (IA): 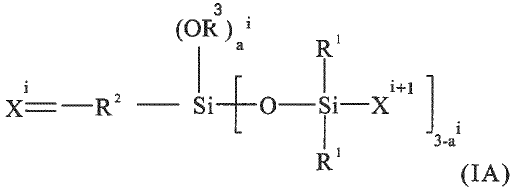 in which:
R¹ is as defined above, R² represents an alkylene group containing from 2 to 10 carbon atoms, R³ represents an alkyl group containing from 1 to 10 carbon atoms, Xⁱ⁺¹ represents a hydrogen atom, an alkyl group containing from 1 to 10 carbon atoms, an aryl group or the silylalkyl group as defined above with i = i + 1; i is an integer ranging from 1 to 10 indicating the generation of the starting silylalkyl group in each carbosiloxane dendritic structure with a value of 1 for the group Xⁱ in formula (IA), and a' is an integer ranging from 0 to 3; Y represents a radical-polymerizable organic group chosen from:
- organic groups containing a methacrylic group or an acrylic group and that are represented by the formulae: 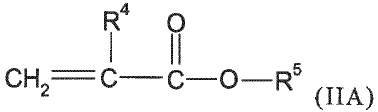 and 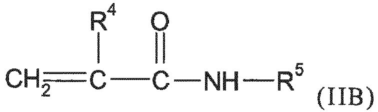 in which R⁴ represents a hydrogen atom or an alkyl group, R⁵ represents an alkylene group containing from 1 to 10 carbon atoms, such as a methylene group, an ethylene group, a propylene group or a butylene group, the methylene group and the propylene group being preferred; and
- organic groups containing a styryl group and that are represented by the formula: 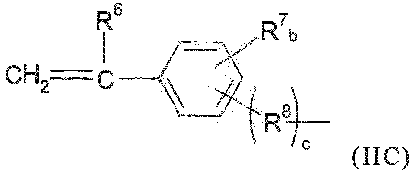 in which R⁶ represents a hydrogen atom or an alkyl group, R⁷ represents an alkyl group containing from 1 to 10 carbon atoms, such as a methyl group, an ethyl group, a propyl group or a butyl group, the methyl group being preferred, R⁸ represents an alkylene group containing from 1 to 10 carbon atoms, such as a methylene group, an ethylene group, a propylene group or a butylene group, the ethylene group being preferred, b is an integer ranging from 0 to 4, and c is 0 or 1 such that if c is 0, -(R⁸)_{c}- represents a bond.

5. Cosmetic composition according to any one of the preceding claims, **characterized in that** the vinyl polymer which has a molecular side chain containing a carbosiloxane dendrimer structure is the product of polymerization of:
- from 0 to 99.9 parts by weight of a vinyl monomer (A) not containing a fluoro organic group,
- from 100 to 0.1 parts by weight of a carbosiloxane dendrimer (B) represented by general formula (I) as defined in Claim 4.

6. Cosmetic composition according to any one of Claims 1 to 5, **characterized in that** the vinyl polymer containing at least one carbosiloxane dendrimer-based unit may contain:
- one or more vinyl monomers (A) not containing a fluoro organic group,
- one or more carbosiloxane dendrimer-based units (B) containing radical-polymerizable organic groups represented by general formula (I) as defined in Claim 4,
- one or more vinyl monomers containing one or more fluoro organic groups (C).

7. Cosmetic composition according to any one of the preceding claims, **characterized in that** the vinyl polymer containing at least one carbosiloxane dendrimer-based unit may comprise tris[tri(trimethylsiloxy)silylethyldimethylsiloxy]silylpropyl carbosiloxane dendrimer-based unit corresponding to one of the following formulae: 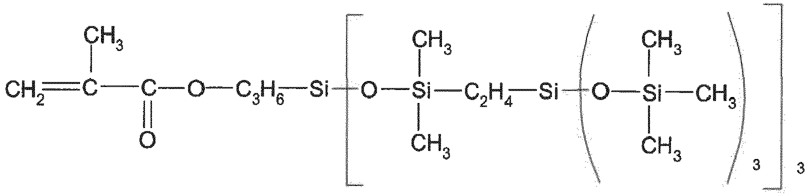 or

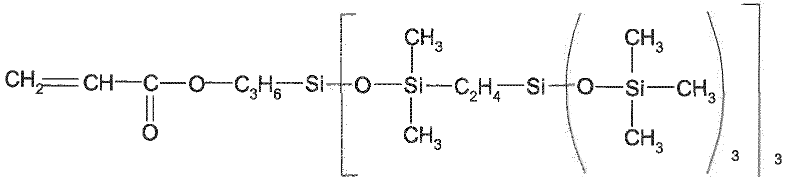

8. Cosmetic composition according to any one of the preceding claims, **characterized in that** the olefin copolymer is chosen from amorphous olefin copolymers or copolymers with controlled and moderate crystallization.

9. Composition according to Claim 8, **characterized in that** the amorphous olefin copolymer is a hydrocarbon-based block copolymer formed by polymerization of ethylenic carbide monomers, in particular containing one or two ethylenic unsaturations, containing from 2 to 5 carbon atoms.

10. Composition according to Claim 9, **characterized in that** the hydrocarbon-based block copolymer is formed by polymerization of an olefin chosen from ethylene, propylene, butadiene and isoprene and more particularly an optionally hydrogenated copolymer, containing styrene blocks and ethylene/C₃-C₄ alkylene blocks.

11. Composition according to Claim 9 or 10, **characterized in that** the hydrocarbon-based block is chosen from optionally hydrogenated styrene-ethylene/propylene, styrene-ethylene/butadiene and styrene-ethylene/butylene diblock copolymers and optionally hydrogenated styrene-ethylene/butadiene-styrene, styrene-butylene/ethylene-styrene, styrene-isoprene-styrene and styrene-butadiene-styrene triblock copolymers and/or a mixture of hydrogenated styrene-butylene/ethylene-styrene triblock copolymer and of styrene-ethylene/butylene diblock copolymer, and more particularly a styrene-ethylene/propylene copolymer.

12. Cosmetic composition according to any one of the preceding claims, **characterized in that** it also comprises one or more emulsifiers chosen from silicone emulsifiers of the alkyl dimethicone copolyol type and of the dimethicone copolyol type, non-silicone W/O emulsifiers with an HLB of 3 to 7, and mixtures thereof.

13. Cosmetic composition according to any one of the preceding claims, **characterized in that** it comprises at least one volatile hydrocarbon-based oil and/or at least one volatile silicone oil, chosen from isododecane, isodecane, isohexadecane, undecane/tridecane mixtures, cyclohexasiloxane, volatile dimethicones, and mixtures thereof.

14. Cosmetic composition according to any one of the preceding claims, **characterized in that** it also comprises one or more polyamide particles, in particular polyamide-12 particles or polyamide-6 particles.

15. Cosmetic composition according to any one of the preceding claims, **characterized in that** it can be packaged in an aerosol device made up of:
(A) a container comprising an antiperspirant composition as defined previously,
(B) at least one propellant and one means for dispensing said aerosol composition.

16. Process for cosmetic treatment of human perspiration, and optionally of underarm odour, consisting in applying an effective amount of the cosmetic composition as defined according to any one of Claims 1 to 15 to the surface of the skin.

17. Treatment process according to Claim 16, **characterized in that** the composition is applied to the surface of the armpits.

## Patentansprüche

1. Kosmetische Antiperspirans-Zusammensetzung in Wasser-in-Öl-Emulsionsform, umfassend einen oder mehrere Antiperspirans-Wirkstoffe, ein oder mehrere Vinylpolymere, die wenigstens eine Einheit auf Carbosiloxandendrimer-Basis umfassen, und ein oder mehrere Olefincopolymere in einem kosmetisch annehmbaren Medium,
wobei die kosmetische Antiperspirans-Zusammensetzung in einer Aerosolform vorliegt.

2. Kosmetische Zusammensetzung gemäß Anspruch 1, **dadurch gekennzeichnet, dass** der Antiperspirans-Wirkstoff ein Aluminiumsalz ist.

3. Kosmetische Zusammensetzung gemäß Anspruch 1 oder 2, **dadurch gekennzeichnet, dass** das Aluminiumsalz ausgewählt ist aus Aluminiumchlorhydrat, Aluminiumchlorhydrex, Aluminiumchlorhydrex-PEG, Aluminiumchlorhydrex-PG, Aluminiumdichlorhydrat, Aluminiumdichlorhydrex-PEG, Aluminiumdichlorhydrex-PG, Aluminiumsesquichlorhydrat, Aluminiumsesquichlorhydrex-PEG, Aluminiumsesquichlorhydrex-PG, Alumsalzen, Aluminiumsulfat, Aluminiumzirkoniumoctachlorhydrat, Aluminiumzirkoniumpentachlorhydrat, Aluminiumzirkoniumtetrachlorhydrat und Aluminiumzirkoniumtrichlorhydrat.

4. Kosmetische Zusammensetzung gemäß einem der Ansprüche 1 bis 3, **dadurch gekennzeichnet, dass** das Vinylpolymer, das wenigstens eine Einheit auf Carbosiloxandendrimer-Basis enthält, eine Molekülseitenkette aufweist, die eine durch die folgende allgemeine Formel (I) dargestellte Carbosiloxan-Dendrimerstruktur enthält: 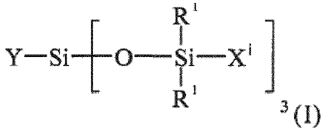 wobei R¹ eine Arylgruppe oder eine Alkylgruppe darstellt, die von 1 bis 10 Kohlenstoffatome enthält, und Xⁱ eine Silylalkylgruppe darstellt, die, wenn i = 1, der Formel (IA) entspricht: 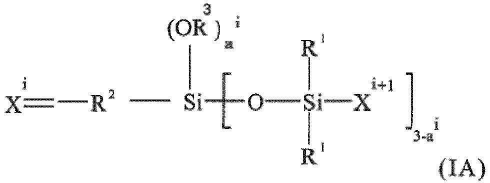 wobei:
R¹ wie vorstehend definiert ist, R² eine Alkylengruppe darstellt, die von 2 bis 10 Kohlenstoffatome enthält, R³ eine Alkylgruppe darstellt, die von 1 bis 10 Kohlenstoffatome enthält, Xⁱ⁺¹ ein Wasserstoffatom, eine Alkylgruppe, die von 1 bis 10 Kohlenstoffatome enthält, eine Arylgruppe oder die wie vorstehend definierte Silylalkylgruppe mit i = i + 1 darstellt; i eine ganze Zahl in dem Bereich von 1 bis 10 ist, die die Generation der Ausgangs-Silylalkylgruppe in jeder dendritischen Carbosiloxanstruktur mit einem Wert von 1 für die Gruppe Xⁱ in Formel (IA) angibt, und aⁱ eine ganze Zahl in dem Bereich von 0 bis 3 ist; Y eine radikalisch polymerisierbare organische Gruppe darstellt, die ausgewählt ist aus:
- organischen Gruppen, die eine Methacrylgruppe oder eine Acrylgruppe enthalten und die durch die Formeln: 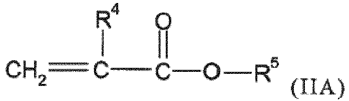 und 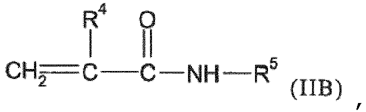 dargestellt werden, wobei R⁴ ein Wasserstoffatom oder eine Alkylgruppe darstellt, R⁵ eine Alkylengruppe darstellt, die von 1 bis 10 Kohlenstoffatome enthält, wie z. B. eine Methylengruppe, eine Ethylengruppe, eine Propylengruppe oder eine Butylengruppe, wobei die Methylengruppe und die Propylengruppe bevorzugt sind; und
- organischen Gruppen, die eine Styrylgruppe enthalten und die durch die Formel: 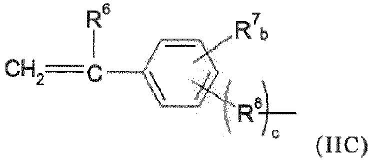 dargestellt werden, wobei R⁶ ein Wasserstoffatom oder eine Alkylgruppe darstellt, R⁷ eine Alkylgruppe darstellt, die von 1 bis 10 Kohlenstoffatome enthält, wie z. B. eine Methylgruppe, eine Ethylgruppe, eine Propylgruppe oder eine Butylgruppe, wobei die Methylgruppe bevorzugt ist, R⁸ eine Alkylengruppe darstellt, die von 1 bis 10 Kohlenstoffatome enthält, wie z. B. eine Methylengruppe, eine Ethylengruppe, eine Propylengruppe oder eine Butylengruppe, wobei die Ethylengruppe bevorzugt ist, b eine ganze Zahl in dem Bereich von 0 bis 4 ist und c 0 oder 1 ist, so dass, wenn c 0 ist, -(R⁸)_{c}- eine Bindung darstellt.

5. Kosmetische Zusammensetzung gemäß einem der vorstehenden Ansprüche, **dadurch gekennzeichnet, dass** das Vinylpolymer, das eine Molekülseitenkette aufweist, die eine Carbosiloxan-Dendrimerstruktur enthält, das Produkt der Polymerisation von:
- von 0 bis 99,9 Gewichtsteilen an einem Vinylpolymer (A), das keine fluororganische Gruppe enthält,
- von 100 bis 0,1 Gewichtsteilen an einem Carbosiloxandendrimer (B), dargestellt durch die allgemeine Formel (I) gemäß Anspruch 4, ist.

6. Kosmetische Zusammensetzung gemäß einem der Ansprüche 1 bis 5, **dadurch gekennzeichnet, dass** das Vinylpolymer, das wenigstens eine Einheit auf Carbosiloxandendrimer-Basis enthält, enthalten kann:
- ein oder mehrere Vinylpolymere (A), die keine fluororganische Gruppe enthalten,
- ein oder mehrere Einheiten auf Carbosiloxandendrimer-Basis (B), die radikalisch polymerisierbare organische Gruppen enthalten, dargestellt durch die allgemeine Formel (I) gemäß Anspruch 4,
- ein oder mehrere Vinylmonomere, die eine oder mehrere fluororganische Gruppen enthalten (C).

7. Kosmetische Zusammensetzung gemäß einem der vorstehenden Ansprüche, **dadurch gekennzeichnet, dass** das Vinylpolymer, das wenigstens eine Einheit auf Carbosiloxandendrimer-Basis enthält, die Einheit auf Carbosiloxan-Dendrimer-Basis Tris[tri(methylsiloxy)silylethyldimethylsiloxy]-silylpropyl gemäß einer der folgenden Formeln: 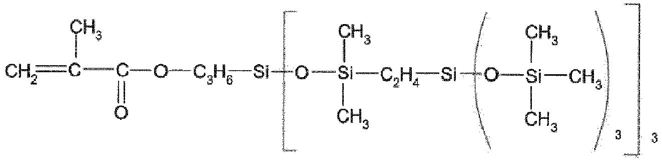 oder 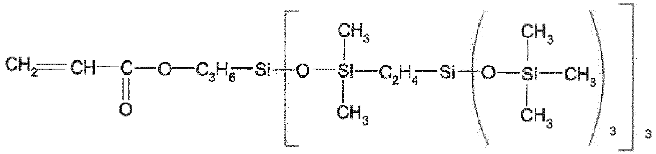 umfassen kann.

8. Kosmetische Zusammensetzung gemäß einem der vorstehenden Ansprüche, **dadurch gekennzeichnet, dass** das Olefincopolymer ausgewählt ist aus amorphen Olefincopolymeren oder Copolymeren mit steuerbarer und mäßiger Kristallisation.

9. Zusammensetzung gemäß Anspruch 8, **dadurch gekennzeichnet, dass** das amorphe Olefincopolymer ein Blockcopolymer auf Kohlenwasserstoffbasis ist, gebildet durch Polymerisation ethylenischer Carbidmonomere, die insbesondere eine oder zwei ethylenische Unsättigungen enthalten und von 2 bis 5 Kohlenstoffatome enthalten.

10. Zusammensetzung gemäß Anspruch 9, **dadurch gekennzeichnet, dass** das Blockcopolymer auf Kohlenwasserstoffbasis durch Polymerisation eines Olefins ausgewählt aus Ethylen, Propylen, Butadien und Isopren gebildet ist, insbesondere ein gegebenenfalls hydriertes Copolymer, das Styrolblöcke und Ethylen/C₃-C₄Alkylenblöcke enthält.

11. Zusammensetzung gemäß Anspruch 9 oder 10, **dadurch gekennzeichnet, dass** der Block auf Kohlenwasserstoffbasis ausgewählt ist aus gegebenenfalls hydrierten Styrol-Ethylen/Propylen-, Styrol-Ethylen/Butadien- und Styrol-Ethylen/Butylen-Diblockcopolymeren und gegebenenfalls hydrierten Styrol-Ethylen/Butadien-Styrol-, Styrol-Butylen/Ethylen-Styrol-, Styrol-Isopren-Styrol- und Styrol-Butadien-Stryrol-Triblockcopolymeren und/oder einem Gemisch von hydriertem Styrol-Butylen/Ethylen-Styrol-Triblockcopolymer und Styrol-Ethylen/Butylen-Diblockcopolymer, insbesondere einem Styrol-Ethylen/Propylen-Copolymer.

12. Kosmetische Zusammensetzung gemäß einem der vorstehenden Ansprüche, **dadurch gekennzeichnet, dass** sie ferner einen oder mehrere Emulgatoren ausgewählt aus Silicon-Emulgatoren vom Alkyldimethicon-Copolyoltyp und vom Dimethicon-Copolyoltyp, Nichtsilicon-W/O-Emulgatoren mit einem HLB-Wert von 3 bis 7 und Gemischen davon umfasst.

13. Kosmetische Zusammensetzung gemäß einem der vorstehenden Ansprüche, **dadurch gekennzeichnet, dass** sie wenigstens ein flüchtiges Öl auf Kohlenwasserstoffbasis und/oder wenigstens ein flüchtiges Siliconöl ausgewählt aus Isododecan, Isodecan, Isohexadecan, Undecan/Tridecan-Gemischen, Cyclohexasiloxan, flüchtigen Dimethiconen und Gemischen davon umfasst.

14. Kosmetische Zusammensetzung gemäß einem der vorstehenden Ansprüche, **dadurch gekennzeichnet, dass** sie ferner ein oder mehrere Polyamidpartikel umfasst, insbesondere Polyamid-12-Partikel oder Polyamid-6-Partikel.

15. Kosmetische Zusammensetzung gemäß einem der vorstehenden Ansprüche, **dadurch gekennzeichnet, dass** sie in einer Aerosolvorrichtung verpackt werden kann, die besteht aus:
(A) einem Behälter, der eine wie vorstehend definierte Antiperspirans-Zusammensetzung umfasst,
(B) wenigstens einem Treibmittel und einem Mittel zum Ausgeben der Aerosolzusammensetzung.

16. Verfahren zur kosmetischen Behandlung von menschlicher Perspiration und gegebenenfalls von Achselgeruch, bestehend aus dem Aufbringen einer wirksamen Menge der kosmetischen Zusammensetzung gemäß einem der Ansprüche 1 bis 15 auf die Oberfläche der Haut.

17. Behandlungsverfahren gemäß Anspruch 16, **dadurch gekennzeichnet, dass** die Zusammensetzung auf die Oberfläche der Achselhöhlen aufgebracht wird.

## Revendications

1. Composition cosmétique anti-transpirante sous forme d'émulsion eau-dans-l'huile comprenant, dans un milieu cosmétiquement acceptable, un ou plusieurs actifs anti-transpirants, un ou plusieurs polymères vinyliques contenant au moins un motif dérivé de dendrimère carbosiloxane et un ou plusieurs copolymères d'oléfine, ladite composition cosmétique anti-transpirante étant sous forme aérosol.

2. Composition cosmétique selon la revendication 1, **caractérisée en ce que** l'actif anti-transpirant est un sel d'aluminium.

3. Composition cosmétique selon la revendication 1 ou 2, **caractérisée en ce que** le sel d'aluminium est choisi parmi le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tétrachlorohydrate, l'aluminium zirconium trichlorohydrate.

4. Composition cosmétique selon l'une quelconque des revendications 1 à 3, **caractérisée en ce que** le polymère vinylique contenant au moins un motif dérivé de dendrimère carbosiloxane possède une chaîne moléculaire latérale contenant une structure de dendrimère carbosiloxane représenté par la formule générale (I) suivante : 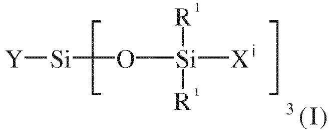 dans laquelle R¹ représente un groupe aryle ou un groupe alkyle comprenant de 1 à 10 atomes de carbone et Xⁱ représente un groupe silylalkyle qui, lorsque i = 1, correspond à la formule (IA) : 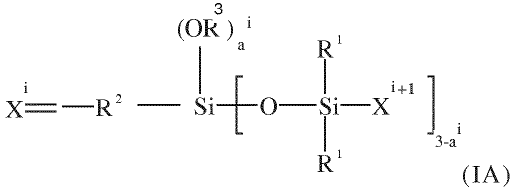 dans laquelle :
R¹ est tel que défini ci-avant, R² représente un groupe alkylène comprenant de 2 à 10 atomes de carbone, R³ représente un groupe alkyle comprenant de 1 à 10 atomes de carbone, Xⁱ⁺¹ représente un atome d'hydrogène, un groupe alkyle possédant de 1 à 10 atomes de carbone, un groupe aryle, ou le groupe silylalkyle tel que défini ci-dessus avec i = i + 1 ; i est un nombre entier allant de 1 à 10 indiquant la génération du groupe silylalkyle commençant dans chaque structure dendritique carbosiloxane avec une valeur de 1 pour le groupement Xⁱ dans la formule (IA) et aⁱ est un nombre entier allant de 0 à 3 ; Y représente un groupe organique polymérisable à l'aide de radicaux choisi parmi :
- des groupes organiques contenant un groupe méthacrylique ou un groupe acrylique et qui sont représentés par les formules : 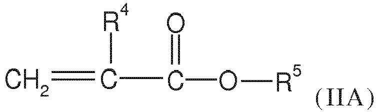 et 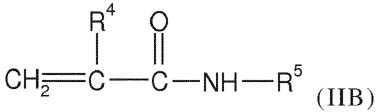 dans lesquelles R⁴ représente un atome d'hydrogène ou un groupe alkyle, R⁵ représente un groupe alkylène possédant de 1 à 10 atomes de carbone tel qu'un groupe méthylène, un groupe éthylène, un groupe propylène, ou un groupe butylène, le groupe méthylène et le groupe propylène étant préférés ; et
- des groupes organiques contenant un groupe styryle et qui sont représentés par la formule : 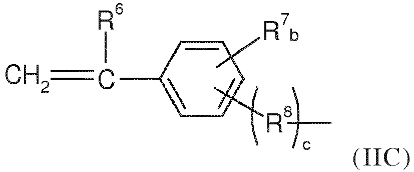 dans laquelle R⁶ représente un atome d'hydrogène ou un groupe alkyle, R⁷ représente un groupe alkyle possédant de 1 à 10 atomes de carbone tel qu'un groupe méthyle, un groupe éthyle, un groupe propyle, ou un groupe butyle, le groupe méthyle étant préféré, R⁸ représente un groupe alkylène possédant de 1 à 10 atomes de carbone tel qu'un groupe méthylène, un groupe éthylène, un groupe propylène, un groupe butylène, le groupe éthylène étant préféré, b est un nombre entier allant de 0 à 4, et c vaut 0 ou 1 de sorte que si c vaut 0, -(R⁸)_{c}-représente une liaison.

5. Composition cosmétique selon l'une quelconque des revendications précédentes, **caractérisée en ce que** le polymère vinylique possédant une chaîne moléculaire latérale contenant une structure de dendrimère carbosiloxane est issu de la polymérisation :
- de 0 à 99,9 parties en poids d'un monomère vinylique (A) ne contenant pas de groupement organique fluoré,
- de 100 à 0,1 parties en poids d'un dendrimère carbosiloxane (B) représenté par la formule générale (I) telle que définie dans la revendication 4.

6. Composition cosmétique selon l'une quelconque des revendications 1 à 5, **caractérisée en ce que** le polymère vinylique contenant au moins un motif dérivé de dendrimère carbosiloxane peut comporter :
- un ou plusieurs monomères vinyliques (A) ne contenant pas de groupement organique fluoré,
- un ou plusieurs motifs dérivés de dendrimère carbosiloxane (B) contenant des groupements organiques polymérisables par voie radicalaire représenté par la formule générale (I) telle que définie dans la revendication 4,
- un ou plusieurs monomères vinyliques contenant un ou plusieurs groupements organiques fluorés (C).

7. Composition cosmétique selon l'une quelconque des revendications précédentes, **caractérisée en ce que** le polymère vinylique contenant au moins un motif dérivé de dendrimère carbosiloxane peut comprendre un motif dérivé de dendrimère carbosiloxane tri[tri(triméthylsiloxy)silyléthyl diméthylsiloxy]silylpropyle répondant à l'une des formules suivantes : 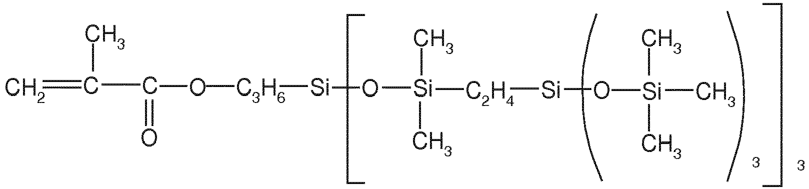 ou

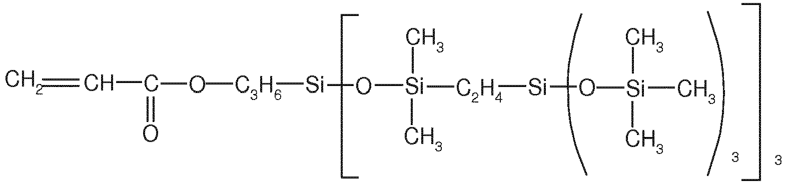

8. Composition cosmétique selon l'une quelconque des revendications précédentes, **caractérisée en ce que** le copolymère d'oléfine est choisi parmi les copolymères d'oléfine amorphes ou à cristallisation contrôlée et modérée.

9. Composition selon la revendication 8, **caractérisée en ce que** le copolymère d'oléfine amorphe est un copolymère bloc hydrocarboné formé par polymérisation de monomères de carbure éthylénique, ayant notamment une ou deux insaturation éthyléniques, ayant de 2 à 5 atomes de carbone.

10. Composition selon la revendication 9, **caractérisée en ce que** le copolymère bloc hydrocarboné est formé par polymérisation d'oléfine choisie parmi l'éthylène, le propylène, le butadiène, l'isoprène et plus particulièrement un copolymère, éventuellement hydrogéné, à blocs styrène et à blocs éthylène/alkylène en C₃-C₄.

11. Composition selon la revendication 9 ou 10, **caractérisée en ce que** le bloc hydrocarboné est choisi parmi les copolymères dibloc, éventuellement hydrogénés, de styrène-éthylène/propylène, de styrène-éthylène/butadiène, de styrène-étylène/butylène et les copolymères tribloc, éventuellement hydrogénés, de styrène-éthylène/butadiène-styrène, de styrène-butylène/éthylène-styrène de styrène-isoprène-styrène, de styrène-butadiène-styrène et/ou un mélange de copolymère hydrogéné tribloc de styrène-butylène/éthylène-styrène et de copolymère dibloc de styrène-étylène/butylène et plus particulièrement un copolymère de styrène-éthylène/propylène.

12. Composition cosmétique selon l'une quelconque des revendications précédentes, **caractérisée en ce qu'**elle comprend en outre un ou plusieurs émulsionnants choisis parmi les émulsionnants siliconés du type alkyldiméthicone copolyol et du type diméthicone copolyol, les émulsionnants E/H non siliconé de HLB 3 à 7 et leurs mélanges.

13. Composition cosmétique selon l'une quelconque des revendications précédentes, **caractérisée en ce qu'**elle comprend au une huile volatile hydrocarbonée et/ou au moins une huile siliconée volatile choisies parmi l'isododécane, l'isodécane, l'isohexadécane, les mélanges undécane/tridécane, la cyclohexasiloxane, les diméthicones volatiles et leurs mélanges.

14. Composition cosmétique selon l'une quelconque des revendications précédentes, **caractérisée en ce qu'**elle comprend en outre une ou plusieurs particules de polyamide, notamment de polyamide-12 ou de polyamide-6.

15. Composition cosmétique selon l'une quelconque des revendications précédentes, **caractérisée en ce qu'**elle peut être conditionnée dans un dispositif aérosol constitué par :
(A) un récipient comprenant une composition anti-transpirante telle que définie précédemment,
(B) au moins un agent propulseur et un moyen de distribution de ladite composition aérosol.

16. Procédé de traitement cosmétique de la transpiration humaine, et éventuellement des odeurs auxiliaires, consistant à appliquer sur la surface de la peau une quantité efficace de la composition cosmétique telle que définie selon l'une quelconque des revendications 1 à 15.

17. Procédé de traitement selon la revendication 16 **caractérisé en ce que** la composition est appliquée sur la surface des aisselles.
